# EUROPÄISCHE PATENTSCHRIFT

(11) **EP 2 451 447 B1**
(45) Veröffentlichungstag und Bekanntmachung des Hinweises auf die Patenterteilung: **11.10.2017**
(21) Anmeldenummer: 10734451.7
(22) Anmeldetag: 07.07.2010
(51) Int. Cl.: A61K 9/20

(54) **WASSERARMES TABLETTIERHILFSMITTEL UND VERFAHREN ZU SEINER HERSTELLUNG**
TABLETING AGENT HAVING A LOW WATER CONTENT, AND METHOD FOR THE PRODUCTION THEREOF
AGENT DE COMPRESSION PAUVRE EN EAU ET SON PROCÉDÉ DE PRÉPARATION

(30) Priorität: 10.07.2009 EP 09009066
(43) Veröffentlichungstag der Anmeldung: 16.05.2012
(73) Patentinhaber: Merck Patent GmbH, 64293 Darmstadt (DE)
(72) Erfinder: EASSON, James, 64289 Darmstadt (DE); HAMM, Walter, 64331 Weiterstadt (DE); MODDELMOG, Guenther, 64354 Reinheim (DE)
(86) Internationale Anmeldenummer: PCT/EP2010/004134
(87) Internationale Veröffentlichungsnummer: WO 2011/003602

(56) Entgegenhaltungen:
- EP-A1- 1 008 353
- EP-A1- 1 674 083
- US-A1- 2004 162 333

## Beschreibung

Die vorliegende Erfindung betrifft ein wasserarmes Tablettierhilfsmittel, gemäß der Definition in den Ansprüchen. Bei der Zusammensetzung des Tablettierhilfsmittels handelt es sich um eine direkt verpressbare Zusammensetzung, die bei ihrer Verwendung zu verbesserten Tabletteneigenschaften führt.

Die Direktverpessung (DC) ist ein einfaches, schnelles, kostengünstiges, Wirkstoff schonendes und flexibles Verfahren in der Tablettenherstellung. Aus verschiedenen Gründen eignen sich jedoch nicht alle zur Formulierung von Tabletten einsetzbaren Komponenten für den Einsatz in diesem Prozess.

So müssen aus Stabilitätsgründen manche feste Darreichungsformen mit besonders wasserarmen Grundstoffen formuliert werden. Mit wasserfreiem Calciumhydrogenphosphat bietet sich hier als solches eine Basissubstanz z. B. für die Herstellung von Tablettenformulierungen an.

Pulverförmiges wasserfreies Calciumhydrogenphosphat kann als Tablettenträgerstoff jedoch wegen schlechter Fließeigenschaften und fehlender Kompressibilität meistens nicht ohne spezielle Zusätze in der Direkttablettierung eingesetzt werden.

In der Regel sind daher nur speziell physikalisch modifizierte wasserfreie Calciumhydrogenphosphate für diesen Prozess geeignet. Aufgrund ihres spröden Materialcharakters ist die Kompressibilität auch dieser Materialien in vielen Rezepturen häufig jedoch nicht ausreichend. Zudem sind die Zerfallszeiten der aus diesen direktverpreßbaren wasserfreien Calciumhydrogenphosphaten hergestellten Preßlinge, auch bedingt durch die geringe Löslichkeit des wasserfreien Calciumhydrogenphospats in wässrigen Medien, teilweise unbefriedigend. Auch organoleptisch weisen wasserfreie DC-Calciumhydrogenphosphate aufgrund der sandigen, scharfkantigen Partikelstruktur und ihrer schlechten Löslichkeit Nachteile auf, sodass ihre Verwendung in oral zerfallenden Darreichungsformen eingeschränkt ist. Darüber hinaus zeigen wasserfreie Calciumhydrogenphosphate im Tablettierungsprozess hohe Ausstoßungskräfte, die zu einer erheblichen mechanischen Beanspruchung der Tablettierwerkzeuge und Maschinen, verbunden mit einem erhöhten Verschleiß der Preßwerkzeuge, aber auch zu einer verstärkten Maschinenbelastung führen, was wiederum zu unerwünschten Ausfallzeiten für Reparaturen oder auch für notwendige Ersatzbeschaffungen führt. Insgesamt wirken sich also diese nachteiligen Eigenschaften negativ auf Haltbarkeit und Laufzeiten der Anlagen aus.

EP 1 674 083 A1 offenbart wirbelschichtgranulierte Zubereitungen, die zu Tabletten verpresst werden und einen Wirkstoff, Mannitol in Kombination mit einem Sorbitol und anorganischer Hilfstoff wie wasserfreies Calciumhydrogenphosphat enthalten. Aufgabe der vorliegenden Erfindung ist es also, ein Verfahren zur Verfügung zu stellen, wodurch auch diese problematischen Wirk- und Hilfsstoffe in einem Verfahren durch Direktverpressen zu Tabletten verarbeitet werden können. Weiterhin ist es Aufgabe der vorliegenden Erfindung, aus diesen Wirk- und Hilfsstoffen frei fließende gut komprimierbare Massen herzustellen, die sich in einfacher Weise unter Vermeidung der oben genannten Nachteile zu Tabletten verpressen lassen.

Die Lösung der vorliegenden Aufgabe erfolgt an sich dadurch, dass man durch die geschickte Kombination und/oder physikalische Modifikation der Hauptbestandteile einer Tabletten rezeptur frei fließende, gut komprimierbare Massen herstellt, die auch unter Zusatz der weniger gut tablettierbaren Rezepturkomponenten eine direkte Verpressung erlauben. Insbesondere werden die Eigenschaften der verschiedenen zugesetzten Komponenten so ausgenutzt, dass diese DC-Materialen einfach zu verarbeiten sind, physiologisch und chemisch inert sind und sich bereits bei möglichst niedrigen Presskräften zu Tabletten mit sehr guten Tablettenhärten bei gleichzeitig ausreichend schnellen Zerfallszeiten verarbeiten lassen.

Gegenstand der vorliegenden Erfindung ist insbesondere eine direkt verpressbare Zusammensetzung für die Tablettenherstellung, bestehend aus einer co-sprühgranulierten Zusammensetzung, die wasserfreies Calciumhydrogenphosphat und ein plastisches Tablettierhilfsmittel in Form von Mannit und Sorbit enthält, worin der Calciumgehalt im Bereich von 14 bis 21 Gew.-% bezogen auf die Gesamtmenge liegt, und die einen Trocknungsverlust kleiner 2 Gew.-%, insbesondere kleiner 1 Gew.-% aufweist.

Diese direkt verpressbare Zusammensetzung für die Tablettenherstellung, besteht insbesondere aus wasserfreiem Calciumhydrogenphosphat und mindestens einem Polyol.

Besonders bevorzugt besteht diese Zusammensetzung aus wasserfreiem Calciumhydrogenphosphat und mindestens einem Polyol, ausgewählt aus der Gruppe Mannit, Sorbit, Xylit und Erythrit. Insbesondere bevorzugt sind in dieser Zusammensetzung wasserfreies Calciumhydrogenphosphat und die Polyole Mannit und Sorbit enthalten.

Besonders gute Eigenschaften haben sich gezeigt, wenn zur Herstellung dieser direkt verpressbaren Zusammensetzung für die Tablettenherstellung eine Kombination aus 50 - 85 Gew.-% wasserfreiem Calciumhydrogenphosphat, 10 - 40 Gew.-% Mannit und 5 - 20 Gew.-% Sorbit, insbesondere eine Kombination, enthaltend 50 bis 80 Gew.-% wasserfreies Calciumhydrogenphosphat , 15 bis 25 Gew.-% Mannit und 7 bis 13 Gew.-% Sorbit, verwendet wird.

Besonders bevorzugt sind entsprechende direkt verpressbare Zusammensetzungen, wenn darin eine Kombination von 65 bis 85 Gew.-% wasserfreiem Calciumhydrogenphosphat, 17 bis 23 Gew.-% Mannit und 8 bis 12 Gew.- % Sorbit enthalten ist.

Insbesondere bevorzugt sind direkt verpressbare Zusammensetzung für die Tablettenherstellung, die aus einer Kombination von 60 bis 80 Gew.-% wasserfreiem Calciumhydrogenphosphat, 15 bis 25 Gew.-% Mannit und 5 bis 15 Gew.-% Sorbit besteht. Vorteilhafte Eigenschaften weisen auch entsprechende Zusammensetzungen auf, in denen wasserfreies Calciumhydrogenphosphat in einer Menge von 65 bis 75 Gew.-%, Mannit in einer Menge von 17 bis 23 Gew.-% und Sorbit in einer Menge von 8 bis 12 Gew.-% enthalten sind und miteinander co-sprühgranuliert worden sind. Erfindungsgemäße direkt verpreßbare, co-sprühgranulierte Zusammensetzungen, wie hier beschrieben, lassen sich sowohl für die Tablettierung als auch zur Kapselherstellung sehr gut dosieren, weil sie einen günstigen Fließwinkel im Bereich von 29 bis 33,4° aufweisen. Da diese Zusammensetzungen Schüttdichten im Bereich von 0,56 bis 0, 77 g/ml und Stampfdichten im Bereich von 0,73 bis 0,92 g/ml besitzen, lassen sie sich besonders gut zu Tabletten mit vergleichsweise hohen Tablettenhärten verarbeiten. In diesem Zusammenhang ist besonders die Kornverteilung in den direkt verpressbaren Zusammensetzungen vorteilhaft, und zwar weisen erfindungsgemäße Zusammensetzungen eine Kornverteilung auf mit max. 3 Gew.-% Unterkorn mit einer Korngröße <32 µm, max. 5 Gew.-% Oberkorn mit einer Korngröße >500 µm, und 50 bis 90 Gew.-% einer Kornfraktion mit Korngrößen im Bereich von 100 bis 315µm. Somit ist auch eine direkt verpressbare Zusammensetzung Gegenstand der vorliegenden Erfindung, die einen Calciumgehalt von 14 bis 21 Gew.-% bezogen auf die Gesamtmenge aufweist, wobei sie einen Trocknungsverlust kleiner 2 Gew.-%, insbesondere kleiner 1 Gew.-% aufweist. In vorteilhafter Weise lassen sich die hier gefundenen, direkt verpressbaren Zusammensetzungen durch Kompression mit einer Preßkraft von 20 kN zu Tabletten mit Härten von > 270 N verpressen, die eine Ausstoßkraft <215 N erfordern, eine Friabilität <0,16 % besitzen, und gleichzeitig eine Zerfallszeit <580 Sekunden zeigen. Insbesondere lassen sie sich durch Kompression mit einer Preßkraft von 20 kN zu Preßlinge mit Härten von >300 N formen, verbunden mit einer Ausstoßkraft von <100 N, welche eine Friabilität von <0,16 % und einer Zerfallszeit von <580 Sekunden besitzen. Bei Erhöhen der Presskraft auf 30 kN werden Preßlinge mit Härten von >350N erhalten, verbunden mit einer Ausstoßkraft von <115N, einer Friabilität von maximal 0,14 % und einer Zerfallszeit von <550 Sekunden. Gegenstand der vorliegenden Erfindung ist somit auch eine Zusammensetzung bzw. Formulierung, welche diese verpressbare Zusammensetzung enthält und als feste Form oder Komprimat vorliegt. Solche eine Zusammensetzung bzw. Formulierung kann einen oder mehrere nicht wasserlösliche und/oder wasserlösliche Zusätze homogen verteilt enthalten. Diese Zusätze sind bevorzugt ausgewählt aus der Gruppe pharmazeutische Wirkstoffe, Pflanzenextrakte, Süßstoffe, Farbstoffe, Zitronensäure, Vitamine und Spurenelemente. Weiterhin kann eine solche erfindungsgemäße Zusammensetzung bzw. Formulierung einen oder mehrere pharmazeutische Wirkstoffe aus der Gruppe der Analgetica enthalten, insbesondere aber auch einen oder mehrere Süßstoffe, ausgewählt aus der Gruppe Acesulfam K, Aspartam^{®}, Saccharin, Cyclamat, Sucralose und Neohesperidin DC.

Die vorliegende Erfindung lässt sich durch ein Verfahren zur Herstellung von direkt verpressbaren Zusammensetzungen für die Tablettenherstellung verwirklichen, worin eine Lösung oder Suspension, enthaltend 50 bis 85 Gew.-% wasserfreies Calciumhydrogenphosphat, 10 bis 40 Gew.-% Mannit und 5 bis 20 Gew.-% Sorbit in Wasser, vorzugsweise 60 bis 80 Gew.-% wasserfreies Calciumhydrogenphosphat, 15 bis 25 Gew.-% Mannit und 7 bis 13 Gew.-% Sorbit in Wasser, wobei in 4 Teilen Wasser 4 Teile Feststoff gelöst, bzw. suspendiert sind, entweder chargenweise oder kontinuierlich in einem Wirbelschichtgranulator einem Co-Sprühgranulationsprozess unterworfen werden.

Durch Versuche wurde gefunden, dass die Kombination des spröden wasserfreien Calciumhydrogenphosphats mit einem im Vergleich dazu eher plastischen Material, wie z.B. einem Polyol, zu einer deutlich verbesserten Tablettenqualität führt, was sich einerseits in einer erheblich verbesserten Kompressibilität zeigt, andererseits aber gleichzeitig Tabletten mit schneller Tablettenzerfallszeit erhalten werden. Insbesondere wurde gefunden, dass aus einer Kombination, bestehend aus einer co-sprühgranulierten Zusammensetzung aus etwa 50 - 85 Gew.-% wasserfreiem pulverförmigem Calciumhydrogenphosphat , etwa 10-40 Gew.-% Mannit und etwa 5 - 20 Gew.-% Sorbit ein entsprechend verbessertes Produkt erhalten werden kann. Insbesondere wird durch einen Co-Sprühgranulationsprozess ein hinsichtlich Fließverhalten, Kompressibilität, Zerfallseigenschaften und anderer galenischer Kennzahlen optimales Produkt für den Direkttablettierungsprozess erhalten. Das erfindungsgemäße Material zeigt deutlich bessere Verarbeitseigenschaften als es z.B. durch einfache physikalische Mischungen selbst unter Verwendung von direkttablettierbaren Einzelkomponenten möglich wäre. Es wurde weiterhin gefunden, dass nur durch Zusatz einer gewissen Menge an Sorbit die galenischen Eigenschaften dieser Co-Sprühprodukte verbessert werden.

Bei der Herstellung von Tabletten ist insbesondere bei gegenüber Feuchtigkeit empfindlichen Wirkstoffen darauf zu achten, dass durch die in einer galenischen Rezeptur eingesetzten Hilfsstoffe möglichst kein Wasser eingebracht wird.

Das Verhältnis der drei oben genannten Bestandteile muss zudem in einem optimierten Bereich gehalten werden, um die verbesserten Preßkraft/Härte-bzw. Härte/Zerfallszeit-Profile zu erhalten. Insbesondere wurde gefunden, dass die verbesserten Eigenschaften erhalten werden, wenn das Gewichtsverhältnis in einem Bereich zwischen etwa 50: 40 : 10 und 70 : 20 : 10 bezogen auf das Verhältnis des eingesetzten wasserfreien Calciumhydrogenphosphats zu Mannit zu Sorbit liegt. In diesem Bereich liefern die entsprechende Zusammensetzungen besonders verbesserte Preßkraft/Härte-bzw. Härte/Zerfallszeit-Profile. Es liegt in dieser Zusammensetzung offensichtlich ein ausgewogenes Verhältnis zwischen der Plastizität der Polyole und der Sprödigkeit des wasserfreien Calciumhydrogenphosphats vor, welches die sehr guten Verpreßeigenschaften bedingt.

Zur Verbesserung der Kompressibilität von wasserfreiem Calciumhydrogenphosphat bei gleichzeitig schneller Tablettenzerfallszeit und möglichst niedrigen Ausstoßungskräften in der Verarbeitung wurde gefunden, dass durch die Kombination des spröden wasserfreien Calciumhydrogenphosphats mit einem im Vergleich dazu eher plastischen Material, wie z. B. einem Polyol, die resultierende Tablettenqualität deutlich verbessert wird.

Insbesondere wurde gefunden, dass durch die Co-Sprühgranulation einer Kombination aus etwa 70 Gew.-% pulverförmigem wasserfreiem Calciumhydrogenphosphat, mit etwa 20 Gew.% Mannit und etwa 10 Gew.% Sorbit ein hinsichtlich Fließverhalten, Kompressibilität, Zerfallseigenschaften und Ausstoßkraft optimales Produkt für den Direkttablettierungsprozess erhalten wird. Beide Polyole besitzen kein Kristallwasser und tragen damit praktisch keine zusätzlichen Wasseranteile in die Formulierung ein. Weiterhin ist es unter den beschrieben Co-Sprühgranulationsbedingungen möglich, wasserarmes Material mit einem Trocknungsverlust <1 Gew.-% zu erhalten. Das erfindungsgemäße Material zeigt deutlich bessere Verarbeitungseigenschaften als sie z.B. durch einfache physikalische Mischungen der entsprechenden wasserarmen Einzelkomponenten erhalten werden könnten. Gegenüber einem verhältnismäßig gut direkttablettierbaren wasserfreien Calciumhydrogenphosphat, das im Handel unter dem Markennamen "Fujicalin" erhältlich ist, weisen die erfindungsgemäß hergestellten Zusammensetzungen verbesserte Eigenschaften auf. So sind bei höheren Preßkräften die Tablettiereigenschaften hinsichtlich Preßkraft/Härte, Härte/Zerfallszeit, und ganz besonders hinsichtlich die notwendigen Ausstoßungskräfte verbessert.

Überraschender Weise wurde gefunden, dass insbesondere unter Sorbitzusatz die galenische Eigenschaften des erhaltenen Co-Sprühproduktes verbessert sind, und zwar insbesondere wenn die oben genannten drei Komponenten in dem optimalen Verhältnis zueinander verwendet werden. Es liegt in dieser Zusammensetzung offensichtlich ein ausgewogenes Verhältnis zwischen Plastizität (der Polyole) und Sprödigkeit (des wasserfreien Calciumhydrogenphosphats) vor, welches die sehr guten Verpreßeigenschaften bedingt.

Ziel der Herstellung der erfindungsgemäßen Zusammensetzungen mit verbesserten Tablettierungseigenschaften muß es also sein, ein Produkt mit einer sehr homogenen Verteilung des in Wasser bei neutralem pH-Wert kaum löslichen wasserfreien Calciumhydrogenphosphats in einer Matrix aus den beiden in Wasser löslichen Polyolen Mannit und Sorbit herzustellen. Diese homogene Verteilung wird, wie die Versuche zeigten, durch ein Co-Sprühgranulationsverfahren aller Komponenten aus wässriger Lösung bzw. Suspension in einem Wirbelbett erzielt.

Um dieses möglichst homogen verteilte wasserfreie Calciumhydrogenphosphat in der Polyolmatrix durch Co-Sprühgranulation zu erhalten, wird beispielsweise in einem Batch-Verfahren zunächst ein Startgranulat erzeugt (Vorsprühung) welches dann im Form einer kleinen Menge zur Vorlage in der Wirbelschicht für einen weiteren oder mehrere Co-Sprühgranulationsprozesse (Hauptsprühungen) dient. Auf diese Weise kann der Anteil an nicht homogen verteilten wasserfreien Calciumhydrogenphosphat in der Polyolmatrix immer weiter bis zu einem vernachlässigbaren Anteil reduziert werden. Eine optimale homogene Verteilung des wasserfreien Calciumhydrogenphosphats in der Polyolmatrix wird idealerweise erhalten, wenn bereits zu Beginn der Co-Sprühgranulation ein Produkt im Wirbelschichtgranulator vorgelegt wird, welches eine homogene Verteilung aufweist. In diesem Fall können die Vorsprühungen entfallen.

In einer kontinuierlichen Fahrweise des Wirbelschichtgranulators wird durch die laufende Entnahme von co-sprühgranuliertem Produkt und eine teilweise Rückführung von gebildetem Produkt der Prozess so geführt, dass nach einer bestimmten Dauer sich der Prozess im Gleichgewicht befindet und das erwünschte homogen verteilte wasserfreie Calciumhydrogenphosphat in der Polyolmatrix erhalten wird. Wenn zu Beginn bereits ein Produkt im Wirbelschichtgranulator vorgelegt wird, welches eine homogene Verteilung aufweist, verkürzt sich selbstverständlich die Dauer für das Anfahren des kontinuierlichen Prozesses bis zum Gleichgewicht.

Mit dem so erhaltenen Material wird dem Galeniker ein hinsichtlich der Direkttablettierungseigenschaften optimiertes Produkt zur Verfügung gestellt, mit dessen Hilfe auch per se schlecht tablettierbare und zudem auch gegenüber Feuchtigkeit empfindliche Wirkstoffe diesem einfachen Tablettierverfahren zugänglich werden können. Durch seinen hohen Calcium- und Phosphatgehalt ist das Produkt außerdem zur Formulierung von mit Calcium und Phosphor angereicherten Preßlingen z.B. in Kautabletten zur Nahrungsergänzung einsetzbar. Diese Verwendung bietet sich insbesondere auch deshalb an, weil das Material durch die Feinstverteilung des von Natur aus sandig schmeckenden wasserfreien Calciumhydrogenphophats in einer Matrix aus den süß und kühl schmeckenden Polyolen sehr gute sensorische Eigenschaften erhält. Es wird somit eine Zusammensetzung zur Verfügung gestellt, das neben der angenehmen Sensorik (Mundgefühl) auch deutlich verbesserte Direktverpressungseigenschaften aufweist. Weiterhin zeigt das Material einen sehr niedrigen Trocknungsverlust.

In diesem Zusammenhang zeigen die erfindungsgemäßen cosprühgranulierten Zusammensetzungen, hergestellt aus wasserfreiem Calciumhydrogenphosphat, Mannit und Sorbit, eine Reihe von unerwarteten Vorteilen:
1. Sehr gute Direktverpreßbarkeitseigenschaften:
   - Während wasserfreies Calciumhydrogenphosphat und Mannit üblicherweise recht schwierig zu Tabletten verpreßt werden können, weist die Dreierkombination wasserfreies Calciumhydrogenphosphat, Mannit und Sorbit nach der Co-Sprühgranulation sehr gute Eigenschaften beim Direktverpressen auf, insbesondere wenn die Komponenten in bestimmten Mengenverhältnissen vorab miteinander einer Co-Sprühgranulation unterzogen werden. Die so erhaltenen Produkte lassen sich anschließend zu Tabletten mit verbesserten Eigenschaften verarbeiten.
   - In den Versuchen wurde bei einem Verhältnis der drei Komponenten zueinander von 70 : 20 : 10, 60 : 30 : 10 und 50 : 40 : 10 die besten Preßkraft-Härte-Relationen erzielt. Bei Kombinationen mit einem Verhältnis der Einzelkomponenten zueinander in diesem Bereich liegen offensichtlich für die Komprimierung optimale Verhältnisse der spröden Eigenschaften des wasserfreien Calciumhydogenphosphats und der plastischen Eigenschaften einer Mannit/Sorbit-Kombination vor. Besonders eine Kombination im Verhältnis von etwa 70 : 20 : 10 zeigt bei höheren Preßkräften die härtesten Komprimate ohne Neigung zum Deckeln und mit geringer Friabilität.
   - Co-Sprühgranulierte Zusammensetzungen mit einem höheren Anteil an wasserfreiem Calciumhydrogenphosphat oder auch solche Zusammensetzungen, in denen wasserfreies Calciumhydrogenphosphat nur mit Mannnit oder nur mit Sorbit co-sprühgranuliert worden ist, zeigen schlechtere Kompressibilitäten.
   - Gegenüber handelsüblichen direktverpreßbaren (DC) wasserfreien Calciumhydrogenphosphat-Qualitäten sind die Direktverpessungseigenschaften der erfindungsgemäßen co-sprühgranulierten Dreierkombinationen verbessert. Dieses gilt ebenso im Vergleich zu einer physikalischen Mischung aus wasserfreiem handelsüblichem DC-Calciumhydrogenphosphat mit dem per se sehr gut direkt verpreßbaren DC-Mannit-Typ (Parteck^{®} M 200) und DC-Sorbit (Parteck^{®} SI 150) im Gewichtsverhältnis von 70 : 20 : 10.
   - lediglich das unter dem Namen Fujicalin^{®} auf dem Markt erhältliche wasserfreie Calciumhydrogenphosphat zeigt ein ähnliches Preßkraft-Härte-Profil, wobei jedoch die erfindungsgemäßen co-sprühgranulierten Zusammensetzungen mit einem Verhältnis der Einzelkomponenten zueinander im Bereich von 60 : 30 : 10 und 70 : 20 : 10 und bei den höheren Preßkräften auch gegenüber Fujicalin^{®} verbesserte Tablettenhärten aufweisen.
2. Schnelle Zerfallszeiten der Preßlinge auch in hohen Härtebereichen
   - Durch die Co-Sprühgranulation als Dreierkombination von 50 bis 85 Gew.-% wasserfreiem Calciumhydrogenphosphat mit bevorzugt mindestens zwei Polyolen, insbesondere bevorzugt Mannit und Sorbit, werden bei gleichzeitig hohen Tablettenhärten auch ohne Zusatz von zerfallsfördenden Komponenten (Sprengmittel) immer noch niedrige Zerfallszeiten erzielt. Ein höherer Zusatz von wasserfreiem Calciumhydrogenphosphat führt zu einer Verzögerung der Zerfallszeit.
   - In den Versuchen zeigten insbesondere die co-sprühgranulierten Dreierkombinationen von wasserfreiem Calciumhydrogenphosphat, Mannit und Sorbit im Gewichtsverhältnis von 70 : 20 : 10 über einen deutlich weiteren Tablettenhärtebereich kurze Zerfallszeiten, und zwar deutlich kürzere als im Handel erhältliches wasserfreies DC-Calciumhydrogenphosphat oder im Vergleich zur physikalischen Mischung der Einzelkomponenten aus DC-Materialen. Lediglich das oben genannte Fujicalin^{®} zeigt kürzere Zerfallszeiten jedoch verbunden mit einem ungünstigerem Preßkraft-/Härteprofil. Insbesondere können mit Fujicalin^{®} nicht die hohen Tablettenhärten aus Beispiel E (70 : 20 : 10) und D (60 : 30 : 10) erreicht werden.
3. Geringer Trockenrückstand
   - Vorteilhafter Weise zeigen erfindungsgemäße Zusammensetzungen geringe Trocknungsverluste von <1 Gew.-% (bei 105 °C für 3 h). Dieses zeigen an, dass auch durch den Co-Sprühgranulationsprozeß kein freies Wasser gebunden wird.
   - Somit sind die erfindungsgemäßen Zusammensetzungen zur Formulierung auch mit gegen Feuchtigkeit empfindliche Wirk- und Hilfsstoffen geeignet.
4. Mechanische Stabilität der resultierenden Tabletten
   Die mechanische Stabilität pharmazeutischen Formulierungen in Form von Granulaten oder Tabletten wird u. a. anhand ihrer Friabilität beurteilt. Friabilität ist das Maß in Gewichtsprozent für den mechanischen Abrieb der Tabletten unter mechanischer Beanspruchung. Von der Herstellung bis zum endgültigen Verbrauch sind Tabletten physischen Belastungen ausgesetzt. Sie müssen daher so entwickelt werden, dass sie die erhaltenen Stöße möglichst unbeschadet überstehen.
   Um festzustellen, wie die zu prüfenden Tabletten diesen Belastungen widerstehen und um Rückschlüsse auf die Weiterverarbeitung (z.B. Lackierung, Dragierung, Konfektionierung) ziehen zu können, werden daher Friabilitäts- oder Abriebtests durchgeführt. Dabei werden die Tabletten nach dem Prinzip einer sich wiederholenden Bewegung in Roche-Friabilator- oder Abriebtrommeln geprüft. Die Testbedingungen, wie Anzahl der Muster, Anzahl der Umdrehungen und - Prüfgeschwindigkeit sind in den Pharmakopöen definiert. Als Abrieb wird die Masse bezeichnet, welche die Tabletten durch die mechanische Belastung verlieren.
   Zur Bestimmung der Friabilität sind im Handel verschiedene Geräte in unterschiedlichen Ausführungsformen erhältlich. Das ERWEKA-Prüfgerät TDR 100 ist ein halbautomatisches Kombinationssystem aus einem ERWEKA Abrieb-/Friabilitätstestgerät und einer Sartorius Analysenwaage (Erweka Apparatebau, Heusenstamm).
   Andere Geräte sind :
   Friabilitätstester Typ TAP Nr. 43651, Erweka Apparatebau, Heusenstamm
   Abriebtester vom Arzneimittelwerk Dresden, Dresden,
   Friabilator Typ PTF 1, Fa. Pharmatest,
   Roche Friabilator, J. Engelsmann AG, Ludwigshafen/Rhein

   Die Friabilität der zu testenden Tabletten wird in diesen Geräten nach Verfahren geprüft, wie in Ph. Eur. Nachtrag 2001 oder Ph. Eur. 6. Ausgabe Grundwerk 2008 unter "2.9.7 Friabilität von nicht überzogenen Tabletten" beschrieben ist.
   Für die Beurteilung kann eine festgelegte Anzahl staubfreier Tabletten in eine Trommel mit einer Schikane für eine bestimmte Zeit und bei festgelegter Drehzahl bewegt werden. Anschließend wird der Masseverlust der von Staub befreiten Tabletten in Prozent bestimmt.
   In den Versuchen der vorliegenden Erfindung wurde die Friabilität der durch Verpressen hergestellten Tabletten bestimmt, indem wie in der Ph. Eur. 6. Ausgabe Grundwerk 2008 unter 2.9.7 beschrieben, Tabletten in einem Friabilator nach Roche auf ihren Abrieb untersucht wurden. Es wurden mit dem Gerät jeweils 100 Umdrehungen durchgeführt, wobei die Umdrehungsgeschwindigkeit bei 25 +/-1 min⁻¹ lag.
   - Die Friabilitäten der erfindungsgemäßen Zusammenssetzungen, bestehend aus der Dreierkombination in einem Gewichtsverhältnis im Bereich zwischen 50 : 40 : 10 und 70 : 20 : 10, insbesondere die untersuchten co-sprühgranulierten Zusammensetzungen im Verhältnis von 50 : 40 : 10, 60 : 30 : 10 und 70 : 20 : 10 sind bei allen geprüften Preßkräften im Vergleich mit im Handel erhältlichen wasserfreien DC-Calciumhydrogenphosphat-Qualitäten wesentlich verringert. Es wird für diese Zusammensetzungen beim Verpressen kein "Deckeln" beobachtet. Dadurch ermöglichen diese Zusammensetzungen eine sehr sichere Handhabung der hergestellten Tabletten, bzw. Preßlinge im weiteren Verarbeitungsprozess, z.B. in Verpackungsmaschinen oder Coatinganlagen, oder bei der Blisterentnahme durch den Patienten. Lediglich verpresste Produkte auf Fujicalin^{®}-Basis zeigen sehr gute Friabilitäten, wobei sie aber nicht die höheren Härten der Beispiele D und E bei den höheren Preßkräften erreichen.
5. Geringe Ausstoßkräfte :
   - Die Ausstoßkräfte für die untersuchten Zusammensetzungen C, D, E, insbesondere für die Zusammensetzung E mit einem Gewichtsverhältnis der Einzelkomponenten zueinander von 70 : 20 : 10, sind bei allen geprüften Preßkräften bei hohen Tablettenhärten verringert im Vergleich zu denen der im Handel erhältlichen direkt verpressbaren wasserfreien Calciumhydrogenphosphate. Dieses führt also zu einer optimale Schonung der Preßwerkzeuge (Stempelwerkzeuge) und der Tablettiermaschinen bei Verwendung der erfindungsgemäßen Zusammensetzungen.
   Insbesondere Fujicalin^{®} zeigt bei allen Preßkräften deutlich erhöhte Ausstoßkräfte, was ein Indikator für eine erhöhte mechanischen Beanspruchung der Tablettierwerkzeuge ist.
   Bei Verwendung erfindungsgemäßer Zusammensetzungen während der Tablettierung klebten diese weder am Stempel noch an den Matrizen der Tablettiermaschine noch zwischen dem Stempel und den Matrizen. Die erfindungsgemäßen Zusammensetzungen neigen auch nicht dazu, sich an den Stempeln und Matrizen anzulagern und dadurch Reibung zwischen dem Stempel und der Matrize zu verursachen. Daher lassen sie sich mit verringertem Druck aus den Tablettiermatrizen ausstoßen.
   Die erfindungsgemäßen Zusammensetzungen lassen sich fortlaufend und stabil für eine lange Zeit auf entsprechenden Tablettiermaschinen industriell verarbeiten, ohne dass es zu einem sogenannten Rauhlaufen der Maschinen kommt.
6. Hoher Calciumhydrogenphosphat-Anteil:
   - Bereits durch die Herstellung der Vorprodukte für die Tablettenherstellung durch Co-Sprühgranulation von wasserfreiem Calciumhydrogenphosphat Mannit und Sorbit in erfindungsgemäßen Gewichtsverhältnissen werden Zusammensetzungen mit einem hohen Calciumhydrogenphosphat-Anteil erhalten, insbesondere durch Co-Sprühgranulation im Gewichtsverhältnis 70 : 20 : 10. Daher können diese Zusammensetzungen zur Calcium- und Phosphoranreichung in Lebensmitteln, Nahrungsergänzungsmittel oder in pharmazeutischen Zubereitungen, insbesondere in Form von Pulversacchets, Preßlingen oder in Kapseln verwendet werden. Weiterhin ist der Fließwinkel des hergestellten Materials im Bereich von 29 bis 33,4° für die Weiterverarbeitung optimal. Das Material ist damit beispielsweise hervorragend zur Einzeldosierung in die Matrizen der Tablettiermaschinen bei der Tablettierung oder zur maschinellen Sacchetabfüllung geeignet.
7. Gute Geschmackseigenschaften:
   Durch die Feinstverteilung des feinen Calciumhydrogenphosphats in der kühlend und süß schmeckenden Polyolmatrix wird das unangenehme sandige Mundgefühl des in Wasser bei neutralem pH-Wert praktisch unlöslichen Calciumhydrogenphosphates überdeckt, so dass durch Verwendung der erfindungsgemäßen Zusammensetzungen die Kunden- respektive die Patientencompliance verbessert wird.
8. Bemerkung zum Fujicalin^{®}:
   Die Versuche haben gezeigt, dass das im Handel erhältliche Fujicalin^{®} in seinen galenischen Eigenschaften den erfindungsgemäßen cosprühgranulierten Zusammensetzungen am nächsten kommt. Jedoch weisen die erfindungsgemäßen Zusammensetzungen wesentliche Verbesserungen gegenüber Fujicalin^{®} auf.
      Fujicalin^{®} weist
      a) wie oben schon gesagt, eine verschlechterten Kompressibilität bei höheren Preßkräften (20 und 30 kN), insbesondere gegenüber Zusammensetzungen der Gewichtsverhältnisse 70 : 20 : 10 (E) und 60 : 30 : 10 (D).
      b) die höchsten Ausstoßkräften gegenüber allen geprüften Muster bei allen geprüften 4 Preßkräften auf auch unter Berücksichtigung der erzielbaren Tablettenhärten.

      Zur Durchführung der Co-Sprühgranulation werden vorab wässrige Lösungen bzw. Suspensionen der verschiedenen Komponenten hergestellt. Bevorzugt wird mit 50-%igen [50%ig(m/m)] Lösungen bzw. Suspensionen gearbeitet, wobei die Prozentangaben sich auf die jeweiligen Gewichtsprozente beziehen. Dabei werden die Trockensubstanzen in vollentsalztem Wasser gelöst, bzw. suspendiert. Zur Durchführung der Co-Sprühganulation werden die mengenmäßigen Verhältnisse des wasserfreien Calciumhydrogenphosphats zu den Polyolen Mannit und Sorbit in Lösung so eingestellt, dass sich die gewünschten gewichtsmäßigen Verhältnisse in der co-gesprühten Substanz im erfindungsgemäßen Verhältnis zueinander befinden. Zur Herstellung der Sprühlösung wird in einem Ansatzgefäß die vorausberechnete Menge vollentsalztes Wasser vorgelegt. In dem Wasser werden unter Rühren bei 20 - 25°C die Polyole Sorbit und Mannit bis zur vollständigen Auflösung eingerührt. In diese klare Lösung wird ebenfalls unter Rühren das Calciumhydrogenphosphat eingetragen und die weiße Suspension solange gerührt bis alle eventuell gebildeten Agglomerate zerfallen sind. Diese Lösung/Suspension wird unter ständigem Rühren in der Co-Sprühgranulation versprüht.
      Um ein möglichst homogen verteiltes Calciumhydrogenphosphat in der Polyolmatrix zu erhalten, kann zunächst in einem Batch-Verfahren ein Startgranulat erzeugt (Vorsprühung) werden, wovon dann jeweils eine kleine Menge zur Vorlage in der Wirbelschicht für einen oder weitere Co-Sprühgranulationsprozesse (Hauptsprühungen) eingesetzt werden kann. Auf diese Weise kann der Anteil an nicht homogen verteiltem Calciumhydrogenphosphat in der Polyolmatrix immer weiter bis zu einem vernachlässigbaren Anteil reduziert werden.
      Wesentlich einfacher ist es natürlich, wenn als Startgranulat co-gesprühtes Material der gewünschten Zusammensetzung eingesetzt wird, welches aus vorhergehenden Sprühungen entnommen worden ist und in der Wirbelschicht vorgelegt werden kann. Die Aufsprühung erfolgt dann wie in den folgenden Beispielen für die Hauptsprühung beschrieben.
      Die kontinuierliche Herstellung wird in ähnlicher Weise durchgeführt wie in den Schriften EP 1 453 781A1, EP 1 319 644 A1 und WO 00/76650 A1 beispielsweise für die Herstellung von alpha- oder beta-Mannit beschrieben. Insbesondere erfolgt die kontinuierliche Herstellung der erfindungsgemäßen Zusammensetzungen in einem Wirbelschichtgranulator mit Pulverrückführung und kontinuierlicher Entnahme von Produkt, wobei die mittlere Korngröße des entstandenen Produkts durch den Luftstrom im Wirbelbett gesteuert wird.
      Durch diese Co-Sprühgranulation wird eine sehr homogene Verteilung des in Wasser bei neutralem pH-Wert kaum löslichen wasserfreien Calciumhydrogenphosphats in einer Matrix aus den beiden in Wasser löslichen Polyolen Mannit und Sorbit erzielt. Diese homogene Verteilung wird durch ein Co-Sprühgranulationsverfahren aller Komponenten aus wässriger Lösung bzw. Suspension in einem Wirbelbett erreicht. Neben einem Produkt mit einer angenehmen Sensorik (Mundgefühl) wird auf diese Weise auch ein Produkt mit sehr guten Direktverpressungseigenschaften erhalten.
      Das Prinzip der Co-Sprühgranulationsverfahrens und die Konstruktion der Anlage ergeben sich aus den Patentschriften EP 1 453 781 (beta-Mannit), EP 1 319 644 (alpha-Mannit) und WO 00/76650.
      Durch Variation der Verfahrensparameter Sprühdruck, Sprühmenge, zurückgeführte Pulvermenge, Heißluftstrom und Temperatur der Heißluft lassen sich die gewünschten Partikelgrößen herstellen. Gegebenenfalls. kann auch durch eine Siebklassierung am Austrag der Anlage eine Korneingrenzung vorgenommen werden. Grobkorn kann über einen Mahlventilator zerkleinert zurück in das Sprühsystem geführt werden.
      Prinzipiell erfolgt die Herstellung der erfindungsgemäßen Zusammensetzungen in ähnlicher Weise wie sie in den Schriften EP 1 453 781A1, EP 1 319 644 A1 für die Herstellung von alpha- und beta-Mannit oder in WO 00/76650 A1 beschrieben ist. Und zwar erfolgt die Co-Sprühgranultion in einem Wirbelschichtgranulator mit Pulverrückführung, worin das Versprühen der Lösungen, bzw. Suspensionen mittels Zweistoffdüsen, über die gleichzeitig rückgeführtes Pulver in die Sprühzone transportiert wird.
      Zu diesem Zweck ist der Sprühdruck der Zweistoffdüsen im Bereich von 2 - 4 bar einzustellen; vorzugsweise wird im Bereich 2,5 - 3,5 bar gearbeitet. Die der Zweistoffdüse zugeführte Menge Heißgas ist so zu regulieren, dass bis zu etwa 1,5 - 3m³/(h kg Suspension) mit einer Temperatur von etwa 80 - 110°C gefördert werden.
      Die Pulverrückführung ist so einzustellen dass eine Feststoffrückführung im Bereich 0,2 - 2,0 kg Feststoff/(h kg Suspension) erfolgt. Vorzugsweise wird im Bereich von 0,5 - 1,5 kg Feststoff /(h kg Lösung) gearbeitet. Besonders günstig gestaltet sich der Prozess, wenn die Feststoffrückführung im Bereich von 0,5 - 1,0 kg/(h kg Lösung) liegt.
      Zur Durchführung des Verfahrens muss in die Anlage vorgewärmte Luft eingespeist werden. Gute Ergebnisse werden erzielt, wenn die der Anlage zugeführte Luft auf eine Temperatur im Bereich von 45 - 120 °C vorgewärmt wird. Günstig ist es für den erfindungsgemäßen Prozess, wenn die Zuluft eine Temperatur im Bereich von 65 - 110 °C aufweist. Besonders vorteilhaft für die Bildung eines co-gesprühgranulierten Calciumhydrogenphosphat/Mannit/Sorbits mit guten Tablettierungseigenschaften ist es, wenn die Temperatur der eingespeisten Zuluft im Bereich von 70 - 100 °C liegt. Die zugeführte Zuluftmenge ist erfindungsgemäß so zu regeln, dass 1000 - 2000 m³/m² pro Stunde, insbesondere 1200 - 1700 m³/m² pro Stunde, in die Anlage eingespeist werden.
      In Verbindung mit den übrigen eingestellten Parametern liegen günstige Verfahrensbedingungen vor, wenn der Luftstrom in der Anlage so geführt wird, dass sich die Ablufttemperatur im Bereich von 30 - 50 °C einstellt und sich die Temperatur des gebildeten Produkts auf eine Temperatur in demselben Bereich bis zu 50 °C einstellt.
      Weiterhin hat es sich als günstig herausgestellt, die Verfahrensbedingungen so zu regulieren, dass sich die im Wirbel- oder Fließbett befindliche Pulvermenge auf eine Bettmenge von 50 - 150 kg/m² Bett einstellt. Besonders günstig ist es, wenn die Bettmenge im Bereich von 80 - 120 kg/m² Bett liegt.
      Durch eine gezielte Pulverrückführung sowohl durch Pulverentnahme aus dem Fließbett als auch durch Rückführung einer sehr feinen Pulverfraktion, welche bei der Konfektionierung d.h. Homogenisierung der Partikelgröße durch Siebung bei Abfüllung des hergestellten Produktes anfällt, lässt sich der Prozeß bezüglich der gewünschten Partikelverteilung steuern.
      Es ist auch möglich, vor der Rückführung vorab Pulver mit größeren Partikelquerschnitten im Mahlventilator der Sprühgranulationseinheit zu zerkleinern, sofern in einem ein Wirbelschichtgranulator gearbeitet wird, wie in EP 1 453 781A1 oder EP 1 319 644 A1 beschrieben ist.
      Durch die besondere Herstellungsweise in einem Co-Sprühgranulationsverfahren werden direkt verpressbare Zusammensetzungen mit einer Schüttdichte im Bereich von 0,56 bis 0, 77 g/ml bei einem Stampfdichte im Bereich von 0,73 bis 0,92 g/ml erhalten. Diese Eigenschaften sind verbunden mit einer Kornverteilung von max. 3 Gew.% Unterkorn mit einer Korngröße <32 µm, von max. 5 Gew.-% Oberkorn mit einer Korngröße >500 µm, und 50 bis 90 Gew.-% einer Kornfraktion mit Korngrößen im Bereich von 100 bis 315µm. Je nach dem Gewichtsanteil von mit versprühten Polyol während der Co-Sprühgranulation besitzt die Zusammensetzung einen Calciumgehalt im Bereich von 14 bis 21 Gew.-% bezogen auf die Gesamtmenge, wobei sie einen Trocknungsverlust kleiner 2 Gew.%, insbesondere kleiner 1 Gew.-% aufweist.
      Durch Untersuchungen der Tablettierungseigenschaften der erfindungsgemäßen direkt verpressbaren Zusammensetzungen wurde gefunden, dass die erfindungsgemäße Zusammensetzung mit einer Preßkraft von 20 kN zu Tabletten mit Härten von > 270 N komprimiert werden kann, verbunden mit einer Ausstoßkraft <215 N, einer Friabilität <0,16 %, einer Zerfallszeit von <580 Sekunden. Wird die erfindungsgemäße Zusammensetzung dagegen mit einer Preßkraft von 30 kN komprimiert, weisen die Preßlinge Härten von >350N auf, verbunden mit einer Ausstoßkraft von <115N, einer Friabilität von maximal 0,14% und einer Zerfallszeit von <550 Sekunden. Durch einen Fließwinkel im Bereich von 29 bis 33,4° lassen sich die erfindungsgemäßen Zusammensetzungen besonders gut in pharmazeutischen Formulierungen Dosieren.
      Erfindungsgemäß kann die direkt verpressbare Zusammensetzung gemäß der vorliegenden Erfindung in eine Zusammensetzung bzw. Formulierung eingebracht werden, welche als feste Form oder Komprimat vorliegt. Als pharmazeutisch einsetzbare Zusammensetzung bzw. Formulierung kann diese wiederum einen oder mehrere nicht wasserlösliche und/oder wasserlösliche Zusätze homogen verteilt enthalten. Die homogene Verteilung kann entweder durch vorheriges intensives Vermischen mit der direkt verpressbaren Zusammensetzung erzeugt werden, bevor die Tablettierung oder Konfektiönierung erfolgt. Die homogene Verteilung kann aber auch erzielt werden durch gemeinsames Co-Sprühgranulieren unter geeigneten Bedingungen. Die wasserlöslichen oder nicht wasserlöslichen Zusätze werden insbesondere ausgewählt aus der Gruppe pharmazeutische Wirkstoffe, Pflanzenextrakte, Süßstoffe, Farbstoffe, Zitronensäure, Vitamine und Spurenelemente. Diese Zusätze werden so ausgewählt, dass sie in der Kombination der Einzelkomponenten der Zusammensetzung stabil und lagerfähig sind. Insbesondere kann eine solche erfindungsgemäße Zusammensetzung bzw. Formulierung einen oder mehrere pharmazeutische Wirkstoffe aus der Gruppe der Analgetica enthalten, weiterhin kann aber auch ein oder mehrere Süßstoffe, ausgewählt aus der Gruppe Acesulfam K, Aspartam^{®}, Saccharin, Cyclamat, Sucralose und Neohesperidin DC, hinzugefügt sein, um den Geschmack zu verbessern.
      Zum besseren Verständnis und zur Verdeutlichung der Erfindung werden im Folgenden Beispiele gegeben, die im Rahmen des Schutzbereichs der vorliegenden Erfindung liegen. Diese Beispiele dienen auch zur Veranschaulichung möglicher Varianten. Aufgrund der allgemeinen Gültigkeit des beschriebenen Erfindungsprinzips sind diese Beispiele jedoch nicht geeignet, den Schutzbereich der vorliegenden Anmeldung nur auf dieses zu reduzieren.
      Zum besseren Verständnis sind, sofern Unklarheiten bestehen, auch die in der Beschreibung zitierten Literaturstellen und Patentschriften heranzuziehen, die hiermit als Teil der Offenbarung in die Beschreibung der vorliegenden Erfindung einbezogen werden.
      Die in den Beispielen und der Beschreibung sowie in den Ansprüchen gegebenen Temperaturen gelten immer in °C. Wo nicht anders bezeichnet, sind Gehaltsangaben als Gew.-% bzw. Gewichtsverhältnisse aufgeführt.
      Weiterhin versteht es sich für den Fachmann von selbst, dass sich sowohl in dem gegebenen Beispiel als auch in der übrigen Beschreibung die in den Zusammensetzungen enthaltenen Komponentenmengen in der Summe immer nur zu 100 Gew. bzw. mol-% bezogen auf die Gesamtzusammensetzung aufaddieren und nicht darüber hinausgehen können, auch wenn sich aus den angegebenen Prozentbereichen höhere Werte ergeben könnten. Sofern nichts anderes angegeben ist, gelten %-Angaben als Gew.-%, mit Ausnahme von Verhältnissen, die in Volumenangaben wiedergegeben sind.

### Beispiele

Zur Durchführung der erfindungsgemäßen Co-Sprühgranulation werden folgende Geräte und Verfahren zur Charakterisierung der Stoffeigenschaften eingesetzt:
1. Schüttdichte: gemäß DIN EN ISO 60: 1999 (Deutsche Fassung)
   - Angabe in den Tabellen als "g/ml"
2. Stampfdichte: gemäß DIN EN ISO 787-11: 1995 (Deutsche Fassung)
   - Angabe in den Tabellen als "g/ml"
3. Schüttwinkel: gemäß DIN ISO 4324: 1983 (Deutsche Fassung)
   - Angabe in den Tabellen als "Grad" (°)
4. Hausner Faktor: Berechnung gemäß Ph.Eur. 6. Ausgabe, Grundwerk 2008, Absatz 2.9.36 "Kompressibilitätsindex und Hausner-Faktor"
5. Kompressibilitätsindex : Berechnung gemäß Ph.Eur. 6.Ausgabe Grundwerk 2008, 2.9.36, "Kompressibilitätsindex und Hausner-Faktor";
   - Angabe in den Tabellen als "%"
6. Tablettierungsprüfung: 492,5 g des auf seine Tablettierungseigenschaften zu prüfenden Materials werden mit 7,5 g Parteck LUB MST (Magnesiumstearat pflanzlich) EMPROVE exp PhEur, BP, JP, NF, FCC Art.No. 1.00663 (Merck KGaA, Deutschland) versetzt; das Magnesiumstearat wird zuvor über ein 250 µm Sieb abgelegt, und 5 Minuten in einem verschlossenen Edelstahlbehälter (Fassungsvolumen: ca. 2 l, Höhe: ca. 19,5 cm, Durchmesser: ca. 12 cm; Außenmaße) auf einem Labor-Taumelmischer (Turbula, Fa. Willy A. Bachofen, Schweiz) gemischt. Die Verpressung zu 500 mg Tabletten (11 mm Stempel, rund, flach, mit Facette) erfolgt auf einer instrumentierten Excenter-Tablettiermaschine Korsch EK 0-DMS (Fa. Korsch, Deutschland) mit dem Auswertesystem Catman 5.0, Fa. Hottinger Baldwin Messtechnik - HBM (Deutschland).
   Je Preßkraft (Soll-Einstellungen:5+/-1, 10+/-1, 20+/-2 und 30+/-2 kN; die effektiv gemessenen Ist-Werte sind in den Beispielen angegeben) werden mindestens 100 Tabletten zur Auswertung der Preßdaten und der galenische Kennzahlen hergestellt.
7. Bestimmung der Tablettenhärte, Durchmesser und Höhen: Erweka TBH 30 MD; Fa. Erweka (Deutschland); Durchschnittsdaten aus jeweils 20 Tablettenmessungen pro Preßkraft
8. Tablettenabrieb: Friabilitätsprüfgerät Fa. Erweka (Deutschland); Geräteparameter und Ausführung der Messungen gemäß Ph.Eur. 6. Ausgabe, Grundwerk 2008, 2.9.7. "Friabilität von nicht überzogenen Tabletten"
9. Tablettenmasse: Durchschnittswert aus der Wägung von 20 Tabletten; Waage: Mettler AT 201, Fa. Mettler (Deutschland)
10. Tablettenzerfall: automatisierter Zerfallstester disi4 der Fa. Biomation (Deutschland); Medium: entsalztes Wasser bei 37°C; Geräteparameter und Ausführung gemäß Ph.Eur. 6. Ausgabe, Grundwerk 2008, 2.9.1 "Zerfallszeit von Tabletten und Kapseln" (mit Scheibe)
11. Bestimmung der Partikelgrößen als Trockensiebung über einen Siebturm Retsch AS 200 control 'g', Fa. Retsch (Deutschland); Substanzmenge: 40g +/- 2g; Siebzeit: 30 Minuten; Amplitude: 1 mm; Intervall: 5 Sekunden; Durchmesser der verwendeten Siebeinsätze: 200 mm; Siebgrößen: 1000, 710, 500, 315, 200, 100, 50 und 32 µm; Angabe der Mengenverteilung pro Siebfraktion in den Tabellen als "Gew.-% der Einwaage"
12. Calciumgehaltsbestimmung: Komplexometrische Titration mit Na-EDTA-Lösung und potentiometrischer Indikation oder Farbindikation. Die prinzipielle Vorgehensweise ist in der Fachliteratur beschrieben wie z.B. in G. Jander, K. F. Jahr, H. Knoll "Maßanalyse - Theorie und Praxis der klassischen und der elektrochemischen Titrierverfahren", Verlag Walter de Gruyter, 1973 ISBN 3 11 005934 7 oder in den Applikationsunterlagen der Titrations- und Indikatorelektrodenhersteller z.B. von der Firma Mettler-Toledo GmbH, Deutschland oder der Firma Metrohm, Schweiz.
   Vor der Titration werden die Proben (ca. 0,2 g Einwaage, genau gewogen) mit etwas vollentsalztem Wasser angeschlämmt und mit 5 ml 25%iger Salzsäure in Lösung gebracht. 20,00 ml Titriplex(III)-Lösung 0,1 mol/L (Art.Nr. 1.08431; MERCK KGaA, Deutschland) werden zudosiert, mit vollentsalztem Wasser auf 70mL aufgefüllt, eine Puffertablette (Art.Nr. 1.08430, MERCK KGaA, Deutschland) hinzugefügt und nach dem Auflösen der Puffertablette unter Rühren mit ca.10 ml Ammoniumpufferlösung pH 10 - 11 (Art.Nr. 1.09478, MERCK KGaA, Deutschland) ein pH-Wert von 10 - 11 eingestellt. Anschließend wird mit einer Zinksulfatlösung (0,1 mol/l) potentiometrisch rücktitriert. Aus der verbrauchten Menge Titripelx(III).Lösung 0,1mol/l kann der Calciumgehalt stöchiometrisch berechnet werden.
13. Trocknungsverlust: Ca. 1,000g Substanz (genau gewogen) werden in einem Trockenschrank 3h bei 105°C getrocknet. Der arithmetische Mittelwert aus zwei unabhängigen Messungen wird als Trocknungsverlust angegeben.

### Rohstoffe zur Herstellung der erfindungsgemäßen Beispiele

Calciumhydrogenphosphat wasserfrei feinst gepulvert, geeignet für die Verwendung als Excipient EMPROVE® exp Ph Eur, BP, USP, FCC, E 341 (Art.Nr. 1.02144, Merck KGaA, Deutschland)
Korngröße: 99%<63µm, gemessen durch Laserbeugung mit Naßdispergierung
Geräte/Methode:
   Malvern Mastersizer 2000, Naßmodul Hydro 2000 S
Probenvorbereitung:
   ca. 500mg Substanz werden in ca. 50 ml wässriger, gesättigter und filtrierter Calciumhydrogenphosphatlösung 1 min im Ultraschallbad dispergiert
Auswertemodel:
   Universal; Medium gesättigte Calciumhydrogenphosphatlösung,
Brechungsindex Medium 1.35 (MIE Parameter); Fraunhofer; Rührgeschwindigkeit 2000 Upm,
Ultraschall:
   100%, Obscuration 10-15%, Messdauer 7500 ms; Ausführung gemäß technischem Handbuch und Spezifikationen des Geräteherstellers
D(-)-Mannit geeignet für die Verwendung als Excipient EMPROVE® exp Ph Eur, BP, USP, JP, FCC, E 321 (Art.Nr. 1.05980, Merck KGaA, Deutschland)
Parteck® SI 400 (Sorbit) geeignet für die Verwendung als Excipient EMPROVE® exp Ph Eur, BP, NF, E 420 (Art.Nr. 1.03140, Merck KGaA, Deutschland)

### Vollentsalztes Wasser

### Vergleichssubstanzen

Parteck^{®} M200 (Mannit) geeignet für die Verwendung als Excipient EMPROVE^{®} exp Ph Eur, BP, JP, USP, E 421 (Art.Nr. 1.00419, Merck KGaA, Deutschland)

Parteck^{®} SI 150 (Sorbit) geeignet für die Verwendung als Excipient EMPROVE^{®} exp Ph Eur, BP, JP, NF, E 420 (Art.Nr. 1.03583, Merck KGaA, Deutschland)

Anhydrous Emcompress^{®} Dibasic Calcium Phosphate, Anhydrous, USP, Calcium Hydrogen Phosphate, Anhydrous, Ph. Eur. (JRS PHARMA GmbH&Co.KG, Deutschland), Batch No: 1046

DI-CAFOS A Dicalcium phosphate anhydrous coarse white powder, USP, FCC, Ph.Eur., JP, E 341 (Product-No.: C 92-12, Chemische Fabrik Budenheim KG, Deutschland)
Material-No. 00000589 Batch-No.: A95505A

A-TAB^{®} Dicalcium Phosphate, Anhydrous, Granular USP, EP, FCC, E 341 (Innophos Inc., USA; bezogen über Univar GmbH, Essen, Deutschland) Lot 2700

DI-CAFOS AN Dicalcium phosphate anhydrous coarse powder, USP, FCC, Ph.Eur., E 341 (Product-No.: C 92-22, Chemische Fabrik Budenheim KG, Deutschland), Material-No. 00005231, Batch-No.: A67665A

FUJICALIN™SG Dibasic Calcium Phosphate Anhydrous DCPA, USP/NF, EP, JP (FULI CHEMICAL INDUSTRY CO., LTD, Japan; bezogen über SEPPIC GmbH, Köln, Deutschland) Lot-No CP 612006

### Allgemeine Durchführung der Co-Sprühgranulation

Eine Lösung bzw. Suspension aus 4 Teilen Wasser und 4 Teilen Feststoff, wobei der Feststoff aus 7 Teilen pulverförmigem, wasserfreiem Calciumhydrogenphosphat, 2 Teilen Mannit und 1 Teil Sorbit besteht, bzw. wobei der Feststoff das Verhältnis der pulverförmigen Ausgangsstoffe wasserfreies Calciumhydrogenphosphat, Mannit und Sorbit in dem gewünschten Verhältnis der herzustellenden Zusammensetzung aufweist, werden in einem Wirbelschichtgranulatör einem Co-Sprühgranulationsprozess (chargenweise oder kontinuierlich) unterworfen. Zur Verhinderung von Klebeeffekten kann insbesondere auch bei Einsatz eines kontinuierlichen Prozesses eine teilweise Feststoffrückführung eingesetzt werden. Durch eine abgestimmte Fahrweise der Anlage bzw. durch ein nachgeschaltetes Siebverfahren kann Produkt einer definierten Kornverteilung bzw. Schütt- und Stampfdichte erhalten werden.

### Co-Sprühganulation im Batch-Betrieb (Labor)

Anmerkung:
Die eingesetzten Rohstoffe, deren Mengen, sowie die quantitativen Zusammensetzungen der co-sprühgranulierten Endprodukte ergeben sich aus den Tabellen.

### Herstellung Sprühlösungen bzw. Sprühsuspensionen:

Alle Sprühlösungen bzw. Suspensionen wurden 50%ig (m/m) an Trockensubstanz in vollentsalztem Wasser hergestellt, wie im allgemeinen Beispiel angegeben. Die Verhältnisse des wasserfreien Calciumhydrogenphosphats zu den Polyolen ergeben sich aus den gewünschten Zusammensetzungen der Gewünschten Endprodukte wie in Tabelle 1 angegeben.

In dem in einem Ansatzgefäß vorgelegten Wasser werden unter Rühren bei 20 - 25 °C die Polyole bis zur vollständigen Auflösung eingerührt. In diese klare Lösung wird, ebenfalls unter Rühren, das wasserfreie Calciumhydrogenphosphat eingetragen und die weiße Suspension solange gerührt bis alle eventuell gebildeten Agglomerate zerfallen sind. Diese Lösung/Suspension wird unter ständigem Rühren versprüht.

### Herstellung des Startmaterials für die Co-Sprühganulation:

Zum Anfahren der Anlage bedarf es einer primären Bettmenge um den Co-Sprühgranulationsprozess in Gang zu bringen. Dieses Startbett kann auf zwei Wegen erzeugt werden:
   1. Man befüllt die Anlage zu Beginn des Sprühprozesses mit zurückgehaltenem aus vorhergehend erfindungsgemäß co-gesprühten Materialmengen
      oder
   2. Man befüllt die Anlage mit einer physikalischen Mischung der gewünschten Komponenten in der herzustellenden qualitativen und quantitativen Zusammensetzung, d.h. mit pulverförmigen wasserfreien Calciumhydrogenphosphat; Mannit und Sorbit. Der Co-Sprühgranulationsprozess wird, wie beschrieben, durchgeführt ohne jedoch Material am Ausgang der Anlage zu entnehmen. Vielmehr wird das Material komplett über den Mahlventilator in den Prozess zurück geführt bis eine stabile Prozessführung und eine erfindungsgemäße Produktzusammensetzung erreicht ist. Danach beginnt die Einstellung der Kornverteilung mit Produktentnahme wie für einen kontinuierliche Prozessführung beschrieben.

Zur Verdeutlichung der entsprechenden Vorgehensweise wird die Durchführung an dem Beispiel beschrieben, welches zur Herstellung der im folgenden als Produkt E benannten co-gesprühten Zusammensetzung führt. Um ein Ausgangsprodukt zu erhalten welches für die Co-Sprühgranulation eingesetzt werden kann, wird durch eine Vorsprühung ein geeignetes Vorprodukt hergestellt, das in dem Wirbelschichtgranulator für die eigentliche Co-Sprühgranulation, die Hauptsprühung, vorgelegt werden kann:

### 1. Vorsprühung:

In 2,20 kg vollentsalztes Wasser werden bei 20 - 25 °C unter Rühren 0,20 kg Mannit und 0,25 kg Sorbit gegeben. Nachdem eine klare Lösung erhalten worden ist, werden unter Rühren 1,75 kg wasserfreies Calciumhydrogenphosphat hinzugegeben und suspendiert.

In einem Wirbelschichtgranulator GPCG 5, Fa.Glatt, Deutschland) oder wie er in WO 00/76650 A1 beschrieben ist, werden 0,3 kg Mannnitpulver vorgelegt und in Verwirbelung gebracht. Auf dieses Wirbelbett wird die Sprühsuspension aufgesprüht. Zur Durchführung der Co-Sprühgranulation werden die Geräteparameter wie im folgenden angegeben eingestellt:
Zuluftklappe ca. 20% (ca. 225m³/h),
Abluftklappe ca. 25%,
Zulufttemperatur ca. 70°C,
Düse: als Zweistoffdüse 1,2 mm im Top down, obere Düsenstellung, Sprühdruck 3,5 bar;
Spührate: steigend von 0,02 kg/Minute auf 0,12 kg/Minute;
Einstellung Ablufttemperatur ca. 40°C
Nach erfolgter Aufsprühung wird das erhaltene Material noch für etwa 10 bis 20 Minuten in der Wirbelschicht nachgetrocknet, wobei die Temperatur der Zuluft so eingestellt wird, dass die Produkttemperatur auf bis zu 50 °C steigt.

### 2. Hauptsprühung:

In 2,50 kg vollentsalztes Wasser werden unter Rühren 0,50 kg Mannit und 0,25 kg Sorbit gelöst. Zu der klaren Lösung werden 1,75 kg wasserfreies Calciumhydrogenphosphat gegeben und suspendiert. Die erhaltene Suspension wird noch für etwa eine Stunde nachgerührt, um alle eventuell entstandenen Agglomerate zu zerstören.

Im Wirbelschichtgranulator (GPCG 5) werden 0,5 kg der Vorsprühung vorgelegt und die Suspension - wie oben anhand der Herstellung der Vorsprühung dargelegt - aufgesprüht.

An diese erste Hauptsprühung können sich mehrere weitere Sprühungen anschließen, wobei bei jeder Sprühung nur jeweils ein geringer Teil der vorhergehenden Sprühung als Wirbelschichtvorlage eingesetzt wird, z. B. wie oben beschrieben 0,5 kg. Auf diese Weise wird der "nicht co-gesprühte" Anteil im Produkt kontinuierlich reduziert.

Die Prüfung der vollständigen Abtrocknung des Produkts erfolgt über die komplexometrische Calcium-Bestimmung sowie über die Bestimmung des Trocknungsverlustes über 3h bei 105°C (als in-process Prüfung). Wesentlich einfacher ist es natürlich wenn als Startgranulat co-gesprühtes Material der gewünschten Zusammensetzung aus vorhergehenden Sprühungen entnommen und in der Wirbelschicht vorgelegt werden kann - die Aufsprühung erfolgt dann wie unter Punkt 2 (Hauptsprühung) beschrieben.

### Versuchsergebnisse:

Die durch die verschiedenen Versuche erzielten Ergebnisse sind in den folgenden Tabellen 1 - 5 wiedergegeben.

In Tabelle 1 sind die getesteten Zusammensetzungen mit unterschiedlichen Gewichtsanteilen an wasserfreiem Calciumhydrogenphosphat, Mannit und Sorbit zusammengefasst.

Tabelle 2 enthält die ermittelten physikalischen Daten der getesteten Zusammensetzungen.

In Tabelle 3 sind die Tablettierungsdaten, Presskraft, Tablettenhärte, Friabilität, Zerfallszeit, Ausstoßkraft, der hergestellten und getesteten Zusammensetzungen zusammengefasst.

In Tabelle 4 sind die entsprechenden physikalischen Daten für handelsübliche wasserfreie und direkt verpreßbare (DC) Calciumhydrogenphosphate und einer tablettierten mechanischen Mischung von wasserfreiem DC-Calciumhydrogenphosphat, DC-Mannit und DC-Sorbit zusammengestellt.

In Tabelle 5 sind den entsprechenden Tablettierungsdaten für handelsübliche wasserfreie DC-Calciumhydrogenphosphate diejenigen der besonders bevorzugten cosprühgranulierten Kombinationen, bestehend aus wasserfreiem Calciumhydrogenphosphat, Mannit und Sorbit der Beispiele C, D und E gegenübergestellt und mit einer entsprechenden mechanischen Mischung verglichen.

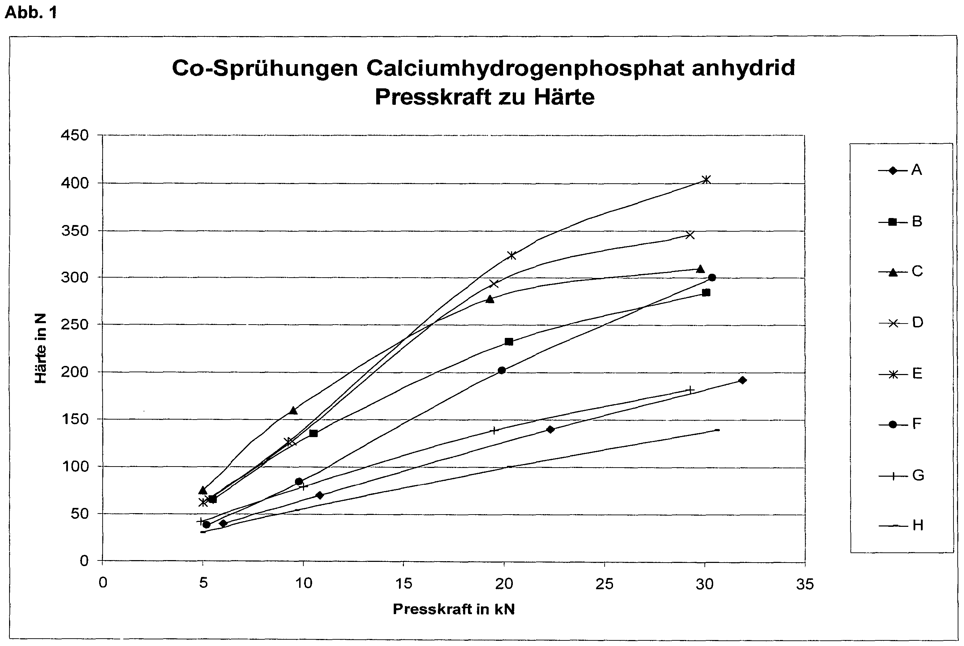

Abb. 1 zeigt die Presskraft Härteprofile für die untersuchten co-gesprühten Zusammensetzungen aus wasserfreiem Calciumhydrogenphosphat, Mannit und Sorbit im Vergleich. Anhand der Profile lässt sich erkennen, dass das Verpressen von einem co-sprühgranulierten, wasserfreien Calciumhydrogenphosphat mit jeweils 5 Gew.-% Mannit und Sorbit (Beispiel H) zu Tabletten mit zunehmendem Preßdruck nur zwischen etwa 30 und 140 N variiert. Wird jedoch eine co-gesprühte Zusammensetzung aus 70 Gew.-% wasserfreiem Calciumhydrogenphosphat und 30 Gew.-% Mannit unter den gleichen Bedingungen mit steigendem Preßdruck tablettiert, werden Produkte mit Härten zwischen etwa 40 und etwa 200 N erhalten. Für die übrigen untersuchten Zusammensetzungen werden Tabletten mit noch höheren Härten unter denselben Bedingungen erhalten. Insbesondere für Zusammensetzungen, in denen das Gew.-Verhältnis von Calciumhydrogenphosphat : Mannit: Sorbit bei 50 : 40 : 10 oder 60 :30 : 10 bzw. 70 : 20 : 10 liegt, werden die höchsten Härten erzielt.

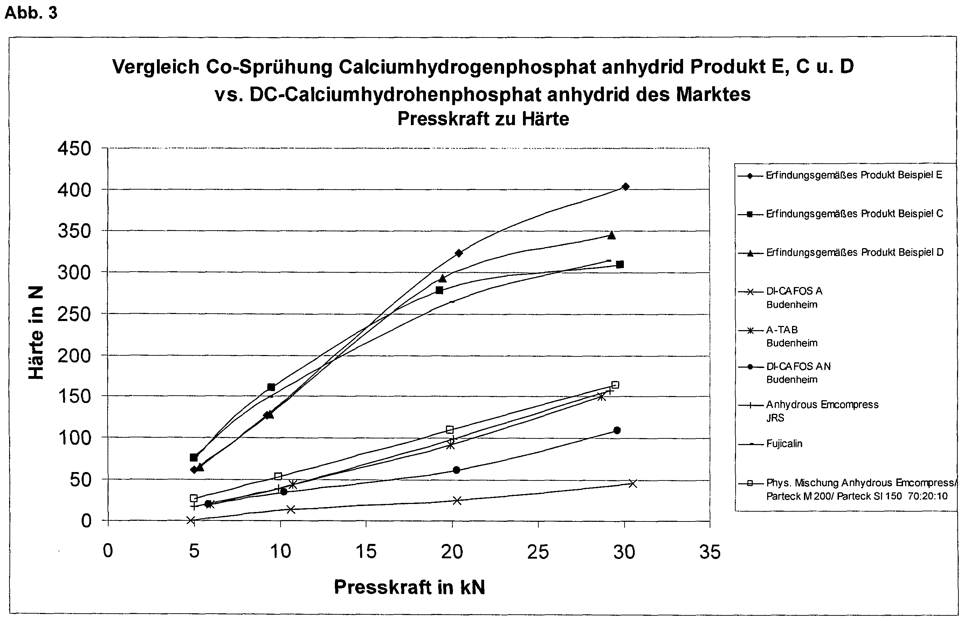

Wie sich in Abb. 3 zeigt, kann durch Vergleich der erfindungsgemäßen Zusammensetzungen mit Tablettiereigenschaften von handelsüblichen direkttablettierfähigen Calciumhydrogenphosphat-anhydrid-Qualitäten festgestellt werden, dass insbesondere für Zusammensetzungen, in denen das Gew.-Verhältnis von Calciumhydrogenphosphat : Mannit : Sorbit bei 50 : 40 : 10, 60 : 30 : 10 bzw. 70 : 20 : 10 liegt, bei gleichen Presskräften erheblich höhere Tablettenhärten erzielt werden ausgenommen von Fujicalin, mit dem vergleichbare Härten erzielt werden wie für die Mischung C, worin Calciumhydrogenphosphat, Mannit und Sorbit im Gewichtsverhältnis von 50 : 40 : 10 miteinander co-sprühgranuliert sind. Hier muss aber angemerkt werden, dass bei Verwendung von Fujicalin bei ähnlicher Tablettenhärte eine deutlich höhere Ausstoßungskraft erforderlich ist als für die Mischung C. Beim Verpressen von physikalischen Mischungen von DC-Calciumhydrogenphosphat anhydrid, DC-Mannit und DC-Sorbit (70 : 20 : 10) werden vergleichsweise ebenso niedrige Tablettenhärten erhalten wie beim Verpressen der handelsüblichen DC-Calciumhydrogenphosphat anhydride.

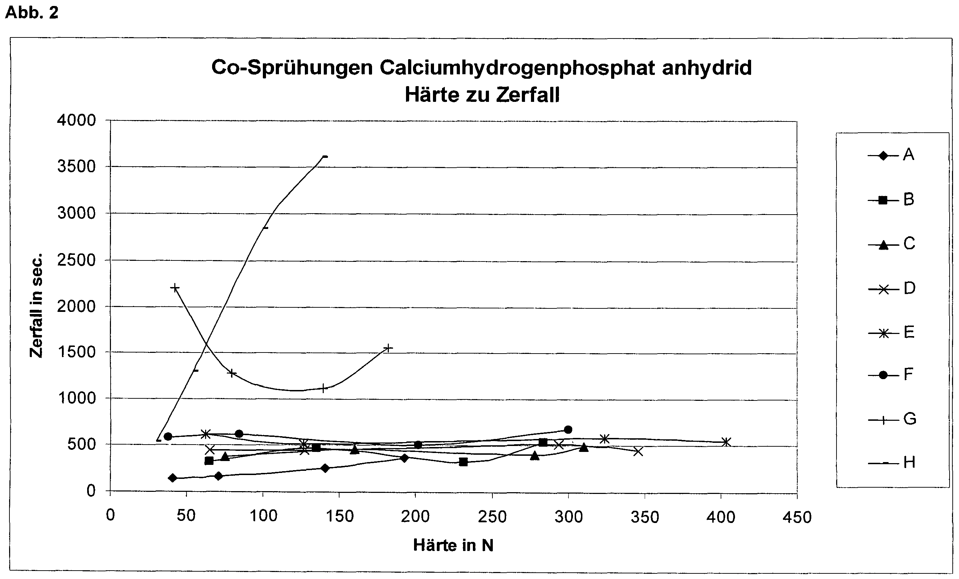

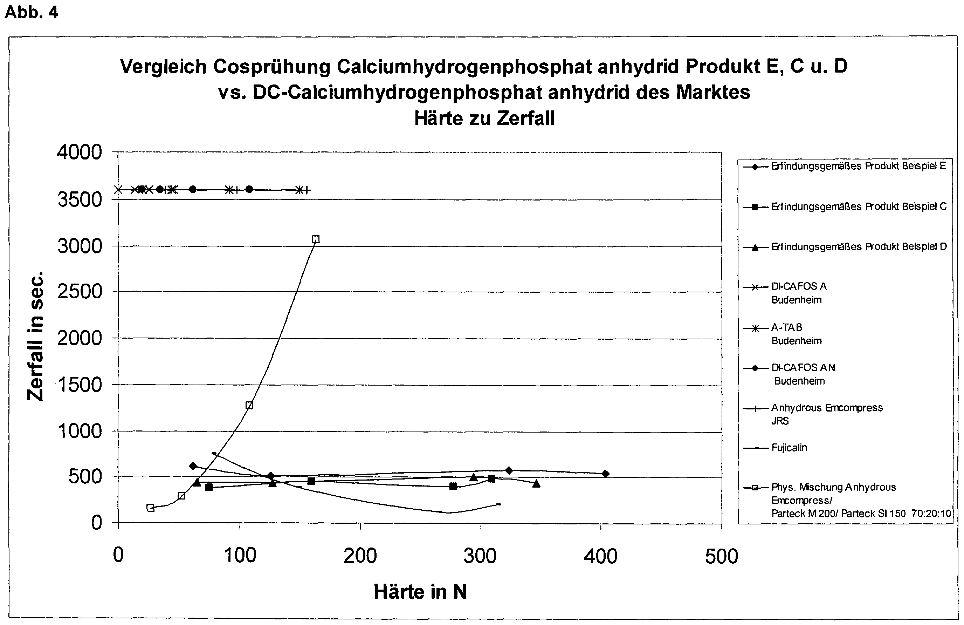

Trotz der erhöhten Tablettenhärten weisen die entsprechenden Tabletten der erfindungsgemäßen Zusammensetzungen sehr kurze Zerfallszeiten im Vergleich zu der Reinsubstanz auf, wie sich aus den graphischen Darstellungen der Abb. 2 und Abb. 4 sehr gut entnehmen lässt. Während eine tablettierte Zusammensetzung, bestehend aus co-sprühgranuliertem Calciumhydrogenphosphat anhydrid, Mannit und Sorbit im Gewichtsverhältnis 90 : 5 : 5, mit zunehmender Härte zwischen 30 und 139 N eine enorm verlängerte Zerfallszeit von bis mehr als 3600 sec zeigt, weisen die erfindungsgemäßen Zusammensetzungen trotz zunehmender Härte lediglich Zerfallszeiten im Bereich von etwa 140 sec bis etwa 670 sec auf, abgesehen von Zerfallszeiten von etwa 1100 bis 2200 sec für verpresste Zusammensetzungen, hergestellt aus einer cosprühgranulierten Zusammensetzung aus 85 Gew.-% Calciumhydrogenphosphat anhydrid und 10 Gew.-% Mannit und 5 Gew.-% Sorbit. Wie aus Abb. 4 zu entnehmen ist, zeigen handelsübliche Produkte im Vergleich zu den erfindungsgemäßen co-sprühgranulierten Zusammensetzungen nach dem Verpressen zu Tabletten mit einer Härte von bis zu 156 N Zerfallszeiten von mehr als 3600 sec auf, mit Ausnahme von Fujicalin, das wie die erfindungsgemäßen Produkte auch bei höheren Tablettenhärten recht niedrige Zerfallszeiten besitzt, wobei jedoch zu berücksichtigen ist, dass es beim Verpressen von Fujicalin zu Tabletten um bis zu fünfmal höhere Ausstoßkräfte notwendig sind im Vergleich zu denen bei Verwendung der erfindungsgemäßen Zusammensetzungen.

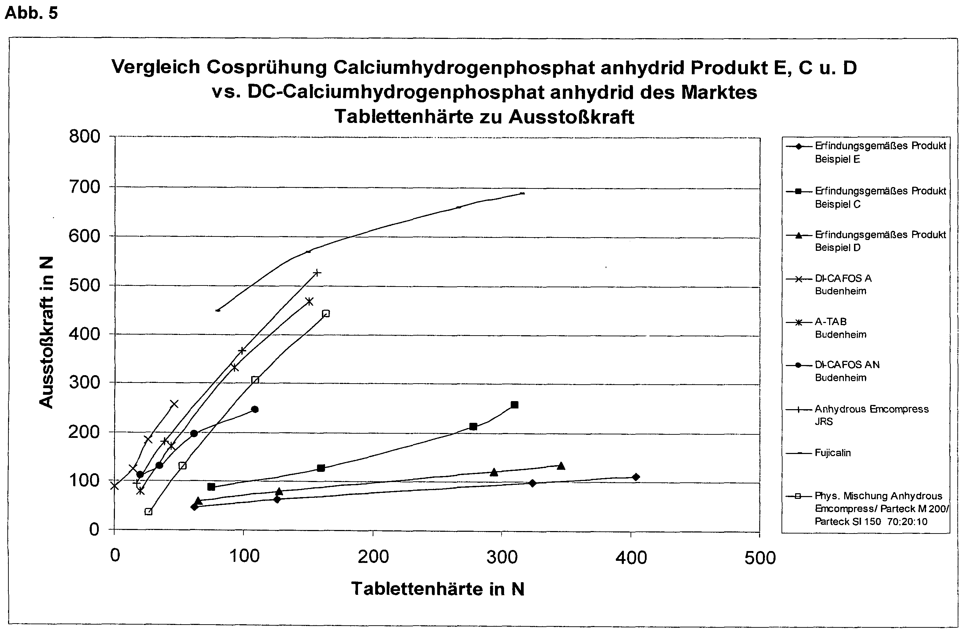

In Abb. 5 sind die Härten der hergestellten Tabletten gegen die Ausstoßungskräfte aufgetragen. Beispielhaft sind dabei die Härten von Tabletten, hergestellt aus Zusammensetzungen der Beispiele C, D und E und den damit verbundenen Ausstoßungskräften mit denen von entsprechenden handelüblichen Produkten verglichen. Dieser Vergleich zeigt recht anschaulich, dass Tabletten aus erfindungsgemäßen Zusammensetzungen trotz zunehmender Härte mit relativ gering zunehmenden Ausstoßungskräften aus den Tablettierwerkzeugen ausgestoßen werden können. Die entsprechenden Vergleichsdaten sind auch aus Tabelle 5 zu entnehmen. Im Gegensatz dazu steigen für die verglichenen handelsüblichen Produkte schon bei recht niedriger Zunahme der Tablettenhärte die erforderlichen Ausstoßkräfte sehr stark an. Dementsprechend ist die Beanspruchung der Tablettiermaschinen wesentlich geringer bei Verwendung der erfindungsgemäßen direkt verpressbaren Zusammensetzungen im Vergleich zur Verwendung von handelsüblichen Zusammensetzungen. Gleiches gilt aber auch für mechanische Mischungen von Calciumphosphat anhydrid mit den Polyolen Mannit und Sorbit im Verhaltnis 70 : 20 : 10. Wie bereits früher angesprochen erfordert besonders in diesem Zusammenhang das im Handel erhältliche Fujicalin nach dem Verpressen besonders hohe Ausstoßkräfte.

**Tabelle 1:**

| Produkt | Calciumhydrogenphosphat anhydrid Art. Nr.: 1.2144 Merck KGaA, Deutschland | Mannit Art. Nr.: 1.05980 Merck KGaA, Deutschland | Sorbit (Parteck SI 400) Art. Nr.: 1.03140 Merck KGaA, Deutschland |
|---|---|---|---|
| A | 70 | 30 | |
| B | 70 | | 30 |
| C | 50 | 40 | 10 |
| D | 60 | 30 | 10 |
| E | 70 | 20 | 10 |
| F | 80 | 15 | 5 |
| G | 85 | 10 | 5 |
| H | 90 | 5 | 5 |

**Tabelle 2:**

| | **A** | **B** | **C** | **D** | **E** | **F** | **G** | **H** |
|---|---|---|---|---|---|---|---|---|
| Schüttdichte [g/ml] | 0,86 | 0,71 | 0,61 | 0,72 | 0,70 | 0,92 | 0,93 | 0,93 |
| Stampfdichte [g/ml] | 1,24 | 0,87 | 0,78 | 0,86 | 0,87 | 1,11 | 1,13 | 1,15 |
| Fließwinkel [°] | 41,1 | 32,6 | 32,6 | 29,8 | 32,0 | 32,3 | 32,1 | 32,1 |
| Hausner Faktor | 1,44 | 1,23 | 1,28 | 1,19 | 1,24 | 1,21 | 1,22 | 1,24 |
| KompressibilitätsIndex [%] | 30,65 | 18,39 | 21,80 | 16,28 | 19,54 | 17,12 | 17,70 | 19,1 3 |
| Kornverteilung (in Gew.-%) | | | | | | | | |
| < 32 µm | 14,07 | 0 | 0,05 | 0,05 | 0,28 | 0,58 | 2,44 | 1,48 |
| 32 - 50 µm | 35,15 | 0,02 | 0,52 | 3,53 | 2,56 | 21,99 | 23,67 | 18,2 4 |
| 50 - 100 µm | 20,51 | 0,76 | 8,83 | 12,53 | 11,13 | 67,81 | 56,33 | 57,1 0 |
| 100 - 200 µm | 24,80 | 3,25 | 32,72 | 23,64 | 59,50 | 7,92 | 12,42 | 15,0 0 |
| 200 - 315 µm | 2,49 | 57,87 | 49,87 | 40,96 | 24,34 | 0,69 | 2,78 | 3,68 |
| 315 - 500 µm | 0,98 | 36,61 | 7,69 | 19,02 | 1,90 | 0,44 | 1,70 | 2,97 |
| 500 - 710 µm | 0,46 | 1,35 | 0,22 | 0,20 | 0,20 | 0,31 | 0,37 | 1,28 |
| 710 - 1000 µm | 0,95 | 0,07 | 0,1 | 0,07 | 0,02 | 0,13 | 0,07 | 0,18 |
| > 1000 µm | 0,59 | 0,07 | 0 | 0 | 0,07 | 0,13 | 0,22 | 0,07 |
| Calciumgehalt [%] | | | | | | | | |
| -theoretisch | 20,6 | 20,6 | 14,7 | 17,7 | 20,6 | 23,6 | 25,0 | 26,5 |
| -gefunden | 20,7 | 20,7 | 14,9 | 17,3 | 20,4 | 23,3 | 25,0 | 26,3 |
| Trocknungsverlust 3 h, 105 °C | 0,37 | 0,13 | 0,33 | 0,36 | 0,20 | 0,25 | 0,30 | 0,36 |

**Tabelle 3:**

| Co-gesprühtes Produkt | Preßkraft [kN] | | Tablettenhärte [N] | Friabilität [%] | Zerfallszeit [sec] | Ausstoßkraft [N] |
|---|---|---|---|---|---|---|
| | soll | ist | | | | |
| A | 5 | 6,0 | 40 | 84,842 | 141 | 87 |
| | 10 | 10,8 | 71 | 0,828 | 163 | 147 |
| | 20 | 22,3 | 140 | 0,290 | 254 | 314 |
| | 30 | 31,9 | 193 | 0,212 | 366 | 441 |
| B | 5 | 5,5 | 65 | 0,706 | 315 | 71 |
| | 10 | 10,5 | 135 | 0,275 | 467 | 106 |
| | 20 | 20,3 | 232 | 0,158 | 313 | 172 |
| | 30 | 30,1 | 284 | 0,130 | 538 | 226 |
| C | 5 | 5,0 | 75 | 0,456 | 376 | 87 |
| | 10 | 9,5 | 160 | 0,202 | 449 | 127 |
| | 20 | 19,3 | 278 | 0,139 | 396 | 213 |
| | 30 | 29,8 | 310 | 0,125 | 486 | 258 |
| D | 5 | 5,3 | 65 | 0,202 | 441 | 60 |
| | 10 | 9,4 | 127 | 0,139 | 445 | 80 |
| | 20 | 19,5 | 294 | 0,123 | 507 | 121 |
| | 30 | 29,3 | 346 | 0,117 | 443 | 134 |
| E | 5 | 5,0 | 62 | 0,212 | 617 | 47 |
| | 10 | 9,2 | 126 | 0,151 | 510 | 64 |
| | 20 | 20,4 | 324 | 0,152 | 574 | 99 |
| | 30 | 30,1 | 404 | 0,134 | 540 | 112 |
| F | 5 | 5,2 | 38 | 0,545 | 581 | 38 |
| | 10 | 9,8 | 84 | 0,200 | 609 | 61 |
| | 20 | 19,9 | 202 | 0,119 | 502 | 105 |
| | 30 | 30,4 | 300 | 0,125 | 671 | 145 |
| G | 5 | 4,9 | 42 | 1,364 | 2200 | 87 |
| | 10 | 10,0 | 79 | 0,470 | 1273 | 113 |
| | 20 | 19,5 | 139 | 0,269 | 1113 | 160 |
| | 30 | 29,3 | 182 | 0,191 | 1560 | 204 |
| H | 5 | 4,9 | 30 | 50,941 | 529 | 29 |
| | 10 | 9,6 | 54 | 0,661 | 1300 | 52 |
| | 20 | 20,2 | 100 | 0,318 | 2846 | 106 |
| | 30 | 30,5 | 139 | 0,208 | >3600 | 159 |

**Tabelle 4:**

| | DI-CAFOS A | A-TAB | DI-CAFOS AN | Anhydrous Emcompress | Fujicalin | Anhydrous Emcompress / Parteck M 200 / Parteck SI 150 70:20:10 mech. Misch. |
|---|---|---|---|---|---|---|
| Schüttdichte (g/ml) | 1,34 | 0,71 | 0,77 | 0,72 | 0,46 | 0,66 |
| Stampfdichte (g/ml) | 1,56 | 0,91 | 0,93 | 0,89 | 0,53 | 0,81 |
| Fließwinkel (°) | 26,2 | 32,3 | 29,1 | 31,8 | 24,7 | 30,6 |
| Hausner Faktor | 1,16 | 1,28 | 1,21 | 1,24 | 1,15 | 1,23 |
| KompressibilitätsIndex (%) | 14,10 | 21,98 | 17,20 | 19,10 | 13,21 | 18,52 |
| Kornverteilung (in Gew.%) | | | | | | |
| < 32 µm | 2,78 | 0,11 | 0 | 0,63 | 0 | 0,72 |
| 32 - 50 µm | 12,98 | 4,14 | 0,15 | 3,59 | 1,55 | 3,37 |
| 50 - 100 µm | 80,03 | 27,37 | 18,28 | 22,38 | 23,40 | 22,09 |
| 100 - 200 µm | 3,16 | 45,60 | 59,16 | 48,11 | 73,26 | 46,36 |
| 200 - 315 µm | 0,53 | 22,27 | 21,95 | 24,63 | 1,74 | 23,20 |
| 315 - 500 µm | 0,32 | 0,45 | 0,38 | 0,52 | 0,05 | 2,94 |
| 500 - 710 µm | 0,16 | 0,06 | 0,08 | 0,12 | 0 | 1,03 |
| 710 - 1000 µm | 0,04 | 0 | 0 | 0,02 | 0 | 0,12 |
| > 1000 µm | 0 | 0 | 0 | 0 | 0 | 0,17 |

**Tabelle 5:**

| Produkt | Presskraft [kN] | | Tablettenhärte [N] | Friabilität [%] | Zerfallszeit [sec.] | Ausstoßkraft [N] |
|---|---|---|---|---|---|---|
| | soll | ist | | | | |
| Erfindungsgemäßes Produkt Bsp. E | 5 | 5 | 62 | 0,212 | 617 | 47 |
| | 10 | 9,2 | 126 | 0,151 | 510 | 64 |
| | 20 | 20,4 | 324 | 0,152 | 574 | 99 |
| | 30 | 30,1 | 404 | 0,134 | 540 | 112 |
| Erfindungsgemäßes Produkt Bsp. C | 5 | 5,0 | 75 | 0,456 | 376 | 87 |
| | 10 | 9,5 | 160 | 0,202 | 449 | 127 |
| | 20 | 19,3 | 278 | 0,139 | 396 | 213 |
| | 30 | 29,8 | 310 | 0,125 | 486 | 258 |
| Erfindungsgemäßes Produkt Bsp. D | 5 | 5,3 | 65 | 0,202 | 441 | 60 |
| | 10 | 9,4 | 127 | 0,139 | 445 | 80 |
| | 20 | 19,5 | 294 | 0,123 | 507 | 121 |
| | 30 | 29,3 | 346 | 0,117 | 443 | 134 |
| DI-CAFOS A Budenheim | 5 | 4,8 | 0 | 100 | >3600 | 90 |
| | 10 | 10,6 | 14 | 100 | >3600 | 125 |
| | 20 | 20,3 | 26 | 100 | >3600 | 185 |
| | 30 | 30,5 | 46 | 65,26 | >3600 | 258 |
| A-TAB Budenheim | 5 | 5,9 | 20 | 1,780 | >3600 | 80 |
| | 10 | 10,7 | 44 | 0,502 | >3600 | 171 |
| | 20 | 19,9 | 92 | 0,275 | >3600 | 332 |
| | 30 | 28,7 | 150 | 0,176 | >3600 | 468 |
| DI-CAFOS AN Budenheim | 5 | 5,8 | 20 | 100 | >3600 | 111 |
| | 10 | 10,2 | 35 | 27,962 | >3600 | 131 |
| | 20 | 20,3 | 62 | 0,968 | >3600 | 196 |
| | 30 | 29,6 | 109 | 0,226 | >3600 | 246 |
| Anhydrous Emcompress JRS | 5 | 5,0 | 17,4 | 36,899 | >3600 | 95 |
| | 10 | 9,9 | 39,2 | 0,571 | >3600 | 181 |
| | 20 | 20,1 | 98,2 | 0,257 | >3600 | 365 |
| | 30 | 29,2 | 156,3 | 0,162 | >3600 | 528 |
| Fujicalin | 5 | 5,0 | 78 | 0,018 | 752 | 448 |
| | 10 | 9,3 | 148 | 0,045 | 394 | 568 |
| | 20 | 19,9 | 265 | 0,021 | 120 | 660 |
| | 30 | 29,0 | 314 | 0,106 | 205 | 688 |
| Phys. Mischung Anhydrous Emcompress / Parteck M200/ Parteck SI150 70:20:10 | 5 | 5,0 | 27 | 1,306 | 151 | 35 |
| | 10 | 9,9 | 53 | 0,365 | 289 | 130 |
| | 20 | 19,9 | 109 | 0,200 | 1274 | 306 |
| | 30 | 29,5 | 164 | 0,168 | 3065 | 442 |

## Patentansprüche

1. Direkt verpressbare Zusammensetzung für die Tablettenherstellung, **dadurch gekennzeichnet, dass** es sich um eine co-sprühgranulierte Zusammensetzung handelt, die aus wasserfreiem Calciumhydrogenphosphat und einem plastischen Tablettierhilfsmittel in Form von Mannit und Sorbit besteht, worin der Calciumgehalt im Bereich von 14 bis 21 Gew.-% bezogen auf die Gesamtmenge liegt, und der Trocknungsverlust kleiner 2 Gew.-%, insbesondere kleiner 1 Gew.-% beträgt.

2. Direkt verpressbare Zusammensetzung nach Anspruch 1 für die Tablettenherstellung, **dadurch gekennzeichnet, dass** sie einen Fließwinkel im Bereich von 29 bis 33,4° aufweist.

3. Direkt verpressbare Zusammensetzung nach Anspruch 1 oder 2 für die Tablettenherstellung, **dadurch gekennzeichnet, dass** sie eine Schüttdichte im Bereich von 0,56 bis 0, 77 g/ml und eine Stampfdichte im Bereich von 0,73 bis 0,92 g/ml aufweist.

4. Direkt verpressbare Zusammensetzung nach einem der Ansprüche 1 bis 3 für die Tablettenherstellung, **dadurch gekennzeichnet, dass** sie eine Kornverteilung aufweist von max. 3 Gew.% Unterkorn mit einer Korngröße <32 µm, von max. 5 Gew.-% Oberkorn mit einer Korngröße >500 µm, und 50 bis 90 Gew.-% einer Kornfraktion mit Korngrößen im Bereich von 100 bis 315µm.

5. Direkt verpressbare Zusammensetzung nach einem der vorhergehenden Ansprüche 1 bis 4, **dadurch gekennzeichnet, dass** die daraus durch Kompression mit einer Preßkraft von 20 kN erhaltenen Tabletten Härten von > 270 N aufweisen, für die eine Ausstoßkraft <215 N ermittelt wird, und die Tabletten eine Friabilität <0,16 % und einer Zerfallszeit <580 Sekunden aufweisen.

6. Direkt verpressbare Zusammensetzung nach einem der vorhergehenden Ansprüche 1 bis 5, **dadurch gekennzeichnet, dass** die daraus durch Kompression mit einer Preßkraft von 20 kN erhaltenen Tabletten Härten von >300 N aufweisen, für die eine Ausstoßkraft von <100 N ermittelt wird, und die Tabletten eine Friabilität von <0,16 % und eine Zerfallszeit von <580 Sekunden aufweisen.

7. Direkt verpressbare Zusammensetzung nach einem der vorhergehenden Ansprüche 1 bis 6, **dadurch gekennzeichnet, dass** die daraus durch Kompression mit einer Preßkraft von 30 kN erhaltenen Tabletten Härten von >350N aufweisen, für die eine Ausstoßkraft von <115N ermittelt wird, und die Tabletten eine Friabilität von maximal 0,14 % und eine Zerfallszeit von <550 Sekunden aufweisen.

8. Zusammensetzung bzw. Formulierung, **dadurch gekennzeichnet, dass** sie eine direkt verpressbare Zusammensetzung nach einem der vorhergehenden Ansprüche 1 bis 7 enthält und als feste Form oder Komprimat vorliegt.

9. Zusammensetzung bzw. Formulierung nach Anspruch 8, **dadurch gekennzeichnet, dass** sie einen oder mehrere nicht wasserlösliche und/oder wasserlösliche Zusätze homogen verteilt enthält.

10. Zusammensetzung bzw. Formulierung nach Anspruch 8 oder 9, **dadurch gekennzeichnet, dass** sie einen oder mehrere Zusätze, ausgewählt aus der Gruppe pharmazeutische Wirkstoffe, Pflanzenextrakte, Süßstoffe, Farbstoffe, Zitronensäure, Vitamine und Spurenelemente enthält.

11. Zusammensetzung bzw. Formulierung nach einem der Ansprüche 8, 9 oder 10, **dadurch gekennzeichnet, dass** sie einen oder mehrere pharmazeutische Wirkstoffe aus der Gruppe der Analgetica enthält.

12. Zusammensetzung bzw. Formulierung nach einem der Ansprüche 8, 9, 10 oder 11, **dadurch gekennzeichnet, dass** sie einen oder mehrere Süßstoffe, ausgewählt aus der Gruppe Acesulfam K, Aspartam^{®}, Saccharin, Cyclamat, Sucralose und Neohesperidin DC enthält.

## Claims

1. Directly compressible composition for the production of tablets, **characterised in that** it is a co-spray-granulated composition which consists of anhydrous calcium hydrogenphosphate and a flexible tabletting assistant in the form of mannitol and sorbitol in which the calcium content is in the range from 14 to 21% by weight, based on the total amount, and the drying loss is less than 2% by weight, in particular less than 1% by weight.

2. Directly compressible composition according to Claim 1 for the production of tablets, **characterised in that** it has a flow angle in the range from 29 to 33.4°.

3. Directly compressible composition according to Claim 1 or 2 for the production of tablets, **characterised in that** it has a bulk density in the range from 0.56 to 0.77 g/ml and a tapped density in the range from 0.73 to 0.92 g/ml.

4. Directly compressible composition according to one of Claims 1 to 3 for the production of tablets, **characterised in that** it has a particle size distribution of max. 3% by weight of fine particles having a particle size < 32 µm, max. 5% by weight of coarse particles having a particle size > 500 µm, and 50 to 90% by weight of a particle fraction having particle sizes in the range from 100 to 315 µm.

5. Directly compressible composition according to one of the preceding Claims 1 to 4, **characterised in that** the tablets obtained therefrom by compression with a pressing force of 20 kN have hardnesses of > 270 N, for which an ejection force of < 215 N is determined, and the tablets have a friability of < 0.16% and a disintegration time of < 580 seconds.

6. Directly compressible composition according to one of the preceding Claims 1 to 5, **characterised in that** the tablets obtained therefrom by compression with a pressing force of 20 kN have hardnesses of > 300 N, for which an ejection force of < 100 N is determined, and the tablets have a friability of < 0.16% and a disintegration time of < 580 seconds.

7. Directly compressible composition according to one of the preceding Claims 1 to 6, **characterised in that** the tablets obtained therefrom by compression with a pressing force of 30 kN have hardnesses of > 350 N, for which an ejection force of < 115 N is determined, and the tablets have a friability of at most 0.14% and a disintegration time of < 550 seconds.

8. Composition or formulation, **characterised in that** it comprises a directly compressible composition according to one of the preceding Claims 1 to 7 and is in solid form or in the form of a compressate.

9. Composition or formulation according to Claim 8, **characterised in that** it comprises one or more homogeneously distributed, water-insoluble and/or water-soluble additives.

10. Composition or formulation according to Claim 8 or 9, **characterised in that** it comprises one or more additives selected from the group pharmaceutical active compounds, plant extracts, sweeteners, dyes, citric acid, vitamins and trace elements.

11. Composition or formulation according to one of Claims 8, 9 or 10, **characterised in that** it comprises one or more pharmaceutical active compounds from the group of the analgesics.

12. Composition or formulation according to one of Claims 8, 9, 10 or 11, **characterised in that** it comprises one or more sweeteners selected from the group acesulfame K, Aspartame^{®}, saccharin, cyclamate, sucralose and neohesperidin DC.

## Revendications

1. Composition directement compressible pour la production de comprimés, **caractérisée en ce qu'**il s'agit d'une composition co-granulée-pulvérisée qui est constituée par de l'hydrogénophosphate de calcium anhydre et par un agent d'aide à la formation de comprimés sous la forme de mannitol et de sorbitol, dans laquelle la teneur en calcium est dans la plage de 14 à 21 % en poids, sur la base de la quantité totale, et la perte au séchage est inférieure à 2 % en poids, en particulier inférieure à 1 % en poids.

2. Composition directement compressible selon la revendication 1 pour la production de comprimés, **caractérisée en ce qu'**elle présente un angle d'écoulement dans la plage de 29 à 33,4°.

3. Composition directement compressible selon la revendication 1 ou 2 pour la production de comprimés, **caractérisée en ce qu'**elle présente une densité apparente dans la plage de 0,56 à 0,77 g/ml et une densité après tassement dans la plage de 0,73 à 0,92 g/ml.

4. Composition directement compressible selon l'une quelconque des revendications précédentes 1 à 3 pour la production de comprimés, **caractérisée en ce qu'**elle présente une distribution de tailles des particules d'au maximum 3 % en poids de particules fines présentant une taille de particule < 32 µm, d'au maximum 5% en poids de grosses particules présentant une taille de particule > 500 µm, et 50 à 90% en poids d'une fraction de particules présentant des tailles de particule dans la plage de 100 à 315 µm.

5. Composition directement compressible selon l'une quelconque des revendications précédentes 1 à 4, **caractérisée en ce que** les comprimés obtenus à partir de celle-ci par compression selon une force de compression de 20 kN présentent des duretés > 270 N, comprimés pour lesquels une force d'éjection < 215 N est déterminée, et les comprimés présentent une friabilité < 0,16% et un temps de désintégration < 580 secondes.

6. Composition directement compressible selon l'une quelconque des revendications précédentes 1 à 5, **caractérisée en ce que** les comprimés obtenus à partir de celle-ci par compression selon une force de compression de 20 kN présentent des duretés > 300 N, comprimés pour lesquels une force d'éjection < 100 N est déterminée, et les comprimés présentent une friabilité < 0,16% et un temps de désintégration < 580 secondes.

7. Composition directement compressible selon l'une quelconque des revendications précédentes 1 à 6, **caractérisée en ce que** les comprimés obtenus à partir de celle-ci par compression selon une force de compression de 30 kN présentent des duretés > 350 N, comprimés pour lesquels une force d'éjection < 115 N est déterminée, et les comprimés présentent une friabilité d'au plus 0,14% et un temps de désintégration < 550 secondes.

8. Composition ou formulation, **caractérisée en ce qu'**elle comprend une composition directement compressible selon l'une quelconque des revendications précédentes 1 à 7 et **en ce qu'**elle est sous forme solide ou sous la forme d'un produit comprimé.

9. Composition ou formulation selon la revendication 8, **caractérisée en ce qu'**elle comprend un ou plusieurs additif(s) distribué(s) de manière homogène, non soluble(s) à l'eau et/ou soluble(s) à l'eau.

10. Composition ou formulation selon la revendication Claim 8 ou 9, **caractérisée en ce qu'**elle comprend un ou plusieurs additif(s) qui est/sont sélectionné(s) parmi le groupe comprenant des composés pharmaceutiques actifs, des extraits de plantes, des édulcorants, des colorants, de l'acide citrique, des vitamines et des oligo-éléments.

11. Composition ou formulation selon l'une des revendications 8, 9 ou 10, **caractérisée en ce qu'**elle comprend un ou plusieurs composé(s) pharmaceutique(s) actif(s) pris parmi le groupe des analgésiques.

12. Composition ou formulation selon l'une des revendications 8, 9, 10 or 11, **caractérisée en ce qu'**elle comprend un ou plusieurs édulcorant(s) qui est/sont sélectionné(s) parmi le groupe comprenant acésulfame K, Aspartame^{®}, saccharine, cyclamate, sucralose et néohespéridine DC.
